(19) Europäisches Patentamt
European Patent Office
Office européen des brevets

(11) **EP 1 764 098 A1**

(12) **EUROPEAN PATENT APPLICATION**

(43) Date of publication:
**21.03.2007 Bulletin 2007/12**

(51) Int Cl.:
***A61K 31/404*** *(2006.01)* ***A61P 25/28*** *(2006.01)*
***A61P 31/18*** *(2006.01)* ***A61P 33/06*** *(2006.01)*

(21) Application number: **06120678.5**

(22) Date of filing: **14.09.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **17.09.2005 CH 15192005**

(71) Applicant: **Speedel Experimenta AG**
**4123 Allschwil (CH)**

(72) Inventors:
- **Herold, Peter**
  **4123, Allschwil (CH)**
- **Mah, Robert**
  **4123, Allschwil (CH)**
- **Stutz, Stefan**
  **4123, Allschwil (CH)**
- **Tschinke, Vincenzo**
  **4123, Allschwil (CH)**
- **Stojanovic, Aleksandar**
  **4123, Allschwil (CH)**
- **Marti, Christiane**
  **4123, Allschwil (CH)**
- **Behnke, Dirk**
  **4123, Allschwil (CH)**
- **Jotterand, Nathalie**
  **4123, Allschwil (CH)**
- **Quirmbach, Michael**
  **4123, Allschwil (CH)**
- **Schumacher, Christoph**
  **4123, Allschwil (CH)**

(74) Representative: **Maué, Paul Georg**
**Solvias AG**
**Patents**
**Erlenstrasse 1**
**4058 Basel (CH)**

(54) **Diaminoalcohols derivatives for the treatment of Alzheimer, malaria, HIV**

(57) Use of compounds of the general formula (I)

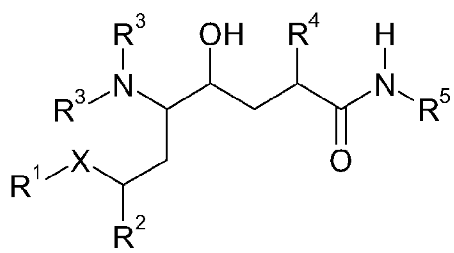

(I)

,

or a pharmaceutically usable salt thereof; wherein $R^1$, $R^2$, $R^3$, $R^4$, $R^5$ and X have the definitions elucidated in more detail in the description, as beta-secretase, cathepsin D, plasmepsin II and/or HIV protease inhibitors.

EP 1 764 098 A1

## Description

The present invention relates to the use of aminoalcohols as beta-secretase-, cathepsin D-, plasmepsin II- and/or HIV-protease-inhibitors.

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neuro-degenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimner's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

**Malaria Aspartyl Protease: Plasmepsin I and II**

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

**HIV aspartyl protease: HIV-1 peptidase**

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated

by an abundance of crystal structure data for both the native enzyme and enzyme-inhibitor complexes. Additionally, there are now extensive biochemical data detailing both the catalytic mechanism and the molecular basis for substrate selection.

Firstly, the present invention relates to the use as beta-secretase-, cathepsin D-, plasmepsin II- and/or HIV-protease-inhibitors of compounds of the general formula

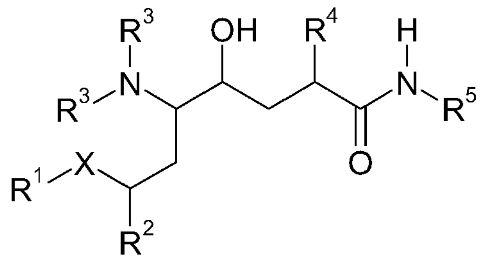

(I)

,

or a pharmaceutically usable salt thereof; wherein

X is $-CH_2-$ ;

(A) $R^1$ is a heterocyclyl radical or a polycyclic, unsaturated hydrocarbon radical which is substituted by one to four radicals selected from $C_1$-$C_6$-alkyl, $C_{3-8}$-cydoalkyl, $C_{3-8}$-cycloalkoxy, $C_{3-8}$-cycloalkoxy-$C_{1-6}$-alkyl, $C_{3-8}$-cycloalkoxy-$C_{1-6}$-alkoxy, $C_1$-$C_6$-alkylamino, di-$C_1$-$C_6$-alkylamino, amino-$C_{1-6}$-alkyl, amino-$C_{2-7}$-alkoxy, polyhalo-$C_{1-6}$-alkyl, polyhalo-$C_{2-7}$-alkoxy, nitro, amino, oxo, oxide, $C_2$-$C_6$-alkenyl, $C_1$-$C_6$-alkoxy, $C_1$-$C_6$-alkanoyloxy, hydroxy, halogen, cyano, carbamoyl, carboxyl, $C_1$-$C_6$-alkylene-dioxy, phenyl, phenoxy, phenylthio, phenyl-$C_1$-$C_6$-alkyl or phenyl-$C_1$-$C_6$-alkoxy, pyridylcarbonylamino-$C_{1-6}$-alkyl, $C_{2-7}$-alkenyloxy, $C_{1-6}$-alkoxy-$C_{1-6}$-alkoxy, $C_{1-6}$-alkoxy-$C_{1-6}$-alkoxy-$C_{1-6}$-alkyl, methoxybenzyloxy, hydroxybenzyloxy, methylenedioxybenzyloxy, dioxolanyl-$C_{1-6}$-alkoxy, $C_{3-8}$-cycloalkyl-$C_{1-6}$-alkyl, $C_{3-8}$-cydoalkyl-$C_{1-6}$-alkoxy, hydroxy-$C_{2-7}$-alkoxy, carbamoyloxy-$C_{2-7}$-alkoxy, pyridylcarbamoyloxy-$C_{2-7}$-alkoxy, benzoyloxy-$C_{2-7}$-alkoxy, $C_{1-6}$-alkoxycarbonyl, $C_{1-6}$-alkylcarbonylamino, $C_{1-6}$-alkylcarbonylamino-$C_{1-6}$-alkyl, $C_{1-6}$-alkylcarbonylamino-$C_{2-7}$-alkoxy, (N-$C_{1-6}$-alkyl)-$C_{1-6}$-alkylcarbonylamino-$C_{1-6}$-alkyl, (N-$C_{1-6}$-alkyl)-$C_{1-6}$-alkylcarbonylamino-$C_{2-7}$-alkoxy, $C_{3-8}$-cycloalkylcarbonylamino-$C_{1-6}$-alkyl, $C_{3-8}$-cycloalkylcarbonylamino-$C_{2-7}$-alkoxy, $C_{1-6}$-alkoxy-$C_{1-6}$-alkyl, hydroxy-$C_{1-6}$-alkyl, hydroxy-$C_{2-7}$-alkoxy-$C_{1-6}$-alkyl, hydroxy-$C_{2-7}$-alkoxy-$C_{1-6}$-alkoxy, $C_{1-6}$-alkoxycarbonylamino-$C_{1-6}$-alkyl, $C_{1-6}$-alkoxycarbonylamino-$C_{2-7}$-alkoxy, $C_{1-6}$-alkylaminocarbonylamino-$C_{1-6}$-alkyl, $C_{1-6}$-alkylaminocarbonylamino-$C_{2-7}$-alkoxy, $C_{1-6}$-alkylaminocarbonyl-$C_{1-6}$-alkyl, $C_{1-6}$-alkylaminocarbonyl-$C_{1-6}$-alkoxy, $C_{1-6}$-alkylaminocarbonyl-$C_{1-6}$-alkoxy-$C_{1-6}$-alkyl, di-$C_{1-6}$-alkylaminocarbonyl-$C_{1-6}$-alkyl, di-$C_{1-6}$-alkylaminocarbonyl-$C_{1-6}$-alkoxy, $C_{1-6}$-alkylcarbonyloxy-$C_{1-6}$-alkyl, $C_{1-6}$-alkylcarbonyloxy-$C_{2-6}$-alkoxy, cyano-$C_{1-6}$-alkyl, cyano-$C_{1-6}$-alkoxy, 2-oxooxazolidinyl-$C_{1-6}$-alkyl, 2-oxooxazolidinyl-$C_{1-6}$-alkoxy, $C_{1-6}$-alkoxycarbonyl-$C_{1-6}$-alkyl, $C_{1-6}$-alkoxycarbonyl-$C_{1-6}$-alkoxy, $C_{1-6}$-alkylsulphonylamino-$C_{1-6}$-alkyl, $C_{1-6}$-alkylsulphonylamino-$C_{1-6}$-alkoxy, (N-$C_{1-6}$-alkyl)-$C_{1-6}$-alkylsulphonylamino-$C_{1-6}$-alkyl, (N-$C_{1-6}$-alkyl)-$C_{1-6}$-alkylsulphonylamino-$C_{2-7}$-alkoxy, $C_{1-6}$-alkylamino-$C_{1-6}$-alkyl, $C_{1-6}$-alkylamino-$C_{2-7}$-alkoxy, di-$C_{1-6}$-alkylamino-$C_{1-6}$-alkyl, di-$C_{1-6}$-alkylamino-$C_{2-7}$-alkoxy, $C_{1-6}$-alkylsulphonyl-$C_{1-6}$-alkyl, $C_{1-6}$-alkylsulphonyl-$C_{1-6}$-alkoxy, carboxy-$C_{1-6}$-alkyl, carboxy-$C_{1-6}$-alkoxy, carboxy-$C_{1-6}$-alkoxy-$C_{1-6}$-alkyl, $C_{1-6}$-alkoxy-$C_{1-6}$-alkylcarbonyl, acyl-$C_{1-6}$-alkoxy-$C_{1-6}$-alkyl, (N-$C_{1-6}$-alkyl)-$C_{1-6}$-alkoxy-carbonylamino, (N-hydroxy)-$C_{1-6}$-alkylaminocarbonyl-$C_{1-6}$-alkyl, (N-hydroxy)-$C_{1-6}$-alkylaminocarbonyl-$C_{1-6}$-alkoxy, (N-hydroxy)aminocarbonyl-$C_{1-6}$-alkyl, (N-hydroxy)aminocarbonyl-$C_{1-6}$-alkoxy, $C_{1-6}$-alkoxyaminocarbonyl-$C_{1-6}$-alkyl, 6-alkoxyaminocarbonyl-$C_{1-6}$-alkoxy, (N-$C_{1-6}$-alkoxy)-$C_{1-6}$-alkylaminocarbonyl-$C_{1-6}$-alkyl, (N-$C_{1-6}$-alkoxy)-$C_{1-6}$-alkylaminocarbonyl-$C_{1-6}$-alkoxy, (N-acyl)-$C_{1-6}$-alkoxy-$C_{1-6}$-alkylamino, $C_{1-6}$-alkoxy-$C_{1-6}$-alkylcarbamoyl, (N-$C_{1-6}$-alkyl)-$C_{1-6}$-alkoxy-$C_{1-6}$-alkylcarbamoyl, $C_{1-6}$-alkoxy-$C_{1-6}$-alkylcarbonyl, $C_{1-6}$-alkoxy-$C_{1-6}$-alkylcarbonylamino, (N-$C_{1-6}$-alkyl)-$C_{1-6}$-alkoxy-$C_{1-6}$-alkylcarbonylamino, 1-$C_{1-6}$-alkoxy-$C_{1-6}$-alkylimidazol-2-yl, 1-$C_{1-6}$-alkoxy-$C_{1-6}$-alkyltetrazol-5-yl, 5-$C_{1-6}$-alkoxy-$C_{1-6}$-alkyltetrazol-1-yl, 2-$C_{1-6}$-alkoxy-$C_{1-6}$-alkyl-4-oxoimidazol-1-yl, carbamoyl-$C_{1-6}$-alkyl, carbamoyl-$C_{1-6}$-alkoxy, $C_{1-6}$-alkylcarbamoyl, di-$C_{1-6}$-alkylcarbamoyl, $C_{1-6}$-alkylsulphonyl, $C_{1-6}$-alkylamidinyl, acetamidinyl-$C_{1-6}$-alkyl, O-methyloximyl-$C_{1-6}$-alkyl, O,N-dimethylhydroxylamino-$C_{1-6}$-alkyl, $C_{3-6}$-cycloalkyl-$C_{1-6}$-alkanoyl, aryl-$C_{1-6}$-alkanoyl or heterocyclyl-$C_{1-6}$-alkanoyl, each of which is optionally substituted by halogen, $C_1$-$C_6$-alkyl, $C_{1-6}$-alkoxy, hydroxy, $C_1$-$C_6$-alkylamino, di-$C_1$-$C_6$-alkylamino, $C_{1-6}$-alkoxycarbonyl, hydroxy-$C_{1-6}$-alkyl or trifluoromethyl, and also pyridyl, pyridyloxy, pyridylthio, pyridylamino, pyridyl-$C_{1-6}$-alkyl, pyridyl-$C_{1-6}$-alkoxy, pyrimidinyl, pyrimidinyloxy, pyrimidinylthio, pyrimidinylamino, pyrimidinyl-$C_{1-6}$-alkyl, pyrimidinyl-$C_{1-6}$-alkoxy, thienyl, thienyl-$C_{1-6}$-alkyl, thienyl-$C_{1-6}$-alkoxy, furyl, furyl-$C_{1-6}$-alkyl or furyl-$C_{1-6}$-alkoxy, piperidi-

noalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-ylalkoxy, 3-methyl[1,2,4]oxadiazol-5-ylalkyl, 3-methyl[1,2,4]oxadiazol-5-ylalkoxy, 5-methyl[1,2,4]oxadiazol-3-ylalkyl, 5-methyl[1,2,4]oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-$C_{1-6}$-alkoxy-$C_{1-6}$-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl, each of which is optionally substituted by halogen, $C_{1-6}$-alkyl, $C_{1-6}$-alkoxy or dihydroxy-$C_{1-6}$-alkylaminocarbonyl, and the -O-$CH_2CH(OH)CH_2NR_x$ radical where $NR_x$ is a mono- or di-$C_{1-6}$-alkylamino, piperidino, morpholino, piperazino or N-methylpiperazino radical, whereby, preferably, in the case that $R^1$ is naphthyl or cyclohexenophenyl, at least the ring ring of said $R^1$ radicals not directly bonded to X is substituted as specified; or

(B) $R^1$ is pyrazinyl, triazolyl, imidazolyl, benzthiazolyl, pyranyl, tetrahydropyranyl, azetidinyl, morpholinyl, quinazolinyl, quinoxalinyl, isoquinolyl, benzo[b]thienyl, isobenzofuranyl, benzimidazolyl, 2-oxobenzimidazolyl, oxazolyl, thiazolyl, pyrrolyl, pyrazolyl, triazinyl, dihydrobenzofuranyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 1,1,3-trioxodihydro-2H-1$\lambda^6$-benzo[1,4]thiazinyl, 1-oxopyridyl, dihydro-3H-benzo-[1,4]oxazinyl, 2-oxotetrahydrobenzo[e][1,4]diazepinyl, 2-oxodihydrobenzo[e][1,4]-diazepinyl, 1H-pyrrolizinyl, phthalazinyl, 1-oxo-3H-isobenzofuranyl, 4-oxo-3H-thieno-[2,3-d]pyrimidinyl, 3-oxo-4H-benzo[1,4]oxazinyl, [1,5]naphthyridyl, dihydro-2H-benzo-[1,4]thiazinyl, 1,1-dioxodihydro-2H-benzo[1,4]thiazinyl, 2-oxo-1H-pyrido[2,3-b][1,4]-oxazinyl, dihydro-1H-pyrido[2,3-b][1,4]oxazinyl, 1 H-pyrrolo[2,3-b]pyridyl, benzo-[1,3]dioxolyl, benzooxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl, or 2-oxotetrahydropyrimidinyl;

$R^2$ is $C_1$-$C_6$-alkyl or $C_3$-$C_6$-cycloalkyl;
$R^3$ are each independently H, $C_1$-$C_6$-alkyl, $C_{1-6}$-alkoxycarbonyl or $C_1$-$C_6$-alkanoyl;
$R^4$ is $C_1$-$C_6$-alkyl, $C_3$-$C_6$-cycloalkyl, $C_2$-$C_6$-alkenyl or unsubstituted or substituted aryl-$C_1$-$C_6$-alkyl; and
$R^5$ is $C_1$-$C_6$-alkyl, $C_1$-$C_6$-hydroxyalkyl, $C_1$-$C_6$-alkoxy-$C_1$-$C_6$-alkyl, $C_1$-$C_6$-alkanoyloxy-$C_1$-$C_6$-alkyl, $C_1$-$C_6$-aminoalkyl, $C_1$-$C_6$-alkylamino-$C_1$-$C_6$-alkyl, $C_1$-$C_6$-dialkylamino-$C_1$-$C_6$-alkyl, $C_1$-$C_6$-alkanoylamido-$C_1$-$C_6$-alkyl, HO(O)C-$C_1$-$C_6$-alkyl, $C_1$-$C_6$-alkyl-O-(O)C-$C_1$-$C_6$-alkyl, $H_2N$-C(O)-$C_1$-$C_6$-alkyl, $C_1$-$C_6$-alkyl-HN-C(O)-$C_1$-$C_6$-alkyl, ($C_1$-$C_6$-alkyl)$_2$N-C(O)-$C_1$-$C_6$-alkyl, $C_2$-$C_8$-alkenyl, $C_2$-$C_8$-alkynyl, cyano-$C_1$-$C_6$-alkyl, halo-$C_1$-$C_6$-alkyl, optionally substituted aryl-$C_0$-$C_6$-alkyl, optionally substituted $C_3$-$C_8$-cycloalkyl-$C_0$-$C_6$-alkyl or optionally substituted heterocyclyl-$C_0$-$C_6$-alkyl.

As alkyl, $R^2$ and $R^4$ may be linear or branched and preferably contain from 1 to 4 carbon atoms. Examples are methyl, ethyl, n- and i-propyl, n-, i- and t-butyl, pentyl and hexyl. In a preferred embodiment, $R^2$ and $R^4$ in the compounds of the formula (I) are each isopropyl.

As alkyl, $R^5$ may be linear or branched and preferably contain from 1 to 4 carbon atoms. Examples of alkyl have been specified above. Preference is given to methyl, ethyl, n- and i-propyl, n-, i- and t-butyl.

As $C_1$-$C_6$-hydroxyalkyl, $R^5$ may be linear or branched and preferably contain from 2 to 6 carbon atoms. Some examples are 2-hydroxyeth-1-yl, 2-hydroxyprop-1-yl, 3-hydroxyprop-1-yl, 2-, 3- or 4-hydroxybut-1-yl, hydroxypentyl and hydroxyhexyl.

As $C_1$-$C_6$-alkoxy-$C_1$-$C_6$-alkyl, $R^5$ may be linear or branched. The alkoxy group preferably contains from 1 to 4 carbon atoms and the alkyl group preferably from 2 to 4 carbon atoms. Some examples are 2-methoxyeth-1-yl, 2-methoxyprop-1-yl, 3-methoxyprop-1-yl, 2-, 3- or 4-methoxybut-1-yl, 2-ethoxyeth-1-yl, 2-ethoxyprop-1-yl, 3-ethoxyprop-1-yl and 2-, 3- or 4-ethoxybut-1-yl.

As $C_1$-$C_6$-alkanoyloxy-$C_1$-$C_6$-alkyl, $R^5$ may be linear or branched. The alkanoyl group contains preferably from 1 to 4 carbon atoms and the alkyl group preferably from 2 to 4 carbon atoms. Some examples are formyloxymethyl, formyloxyethyl, acetyloxyethyl, propionyloxyethyl and butyryloxyethyl.

As $C_1$-$C_6$-aminoalkyl, $R^5$ may be linear or branched and preferably contain from 2 to 4 carbon atoms. Some

examples are 2-aminoethyl, 2- or 3-aminoprop-1-yl and 2-, 3- or 4-aminobut-1-yl.

As $C_1$-$C_6$-alkylamino-$C_1$-$C_6$-alkyl and $C_1$-$C_6$-dialkylamino-$C_1$-$C_6$-alkyl, $R^5$ may be linear or branched. The alkylamino group contains preferably $C_1$-$C_4$-alkyl groups and the alkyl group preferably from 2 to 4 carbon atoms. Some examples are 2-methylaminoeth-1-yl, 2-dimethyl-aminoeth-1-yl, 2-ethylaminoeth-1-yl, 2-ethylaminoeth-1-yl, 3-methylaminoprop-1-yl, 3-dimethylaminoprop-1-yl, 4-methylaminobut-1-yl and 4-dimethylaminobut-1-yl.

As $C_1$-$C_6$-alkanoylamido-$C_1$-$C_6$-alkyl, $R^5$ may be linear or branched. The alkanoyl group contains preferably from 1 to 4 carbon atoms and the alkyl group preferably from 1 to 4 carbon atoms. Some examples are 2-formamidoeth-1-yl, 2-acetamidoeth-1-yl, 3-propionyl-amidoeth-1-yl and 4-butyrylamidoeth-1-yl.

As HO(O)C-$C_1$-$C_6$-alkyl, $R^5$ may be linear or branched, and the alkyl group preferably contains from 2 to 4 carbon atoms. Some examples are carboxymethyl, carboxyethyl, carboxypropyl and carboxybutyl.

As $C_1$-$C_6$-alkyl-O-(O)C-$C_1$-$C_6$-alkyl, $R^5$ may be linear or branched, and the alkyl groups preferably each independently contain from 1 to 4 carbon atoms. Some examples are methoxycarbonylmethyl, 2-methoxycarbonyleth-1-yl, 3-methoxycarbonylprop-1-yl, 4-methoxycarbonylbut-1-yl, ethoxycarbonylmethyl, 2-ethoxycarbonyleth-1-yl, 3-ethoxycarbonylprop-1-yl, 4-ethoxycarbonylbut-1-yl.

As $H_2N$-C(O)-$C_1$-$C_6$-alkyl, $R^5$ may be linear or branched, and the alkyl group preferably contains from 2 to 6 carbon atoms. Some examples are carbamidomethyl, 2-carbamidoeth-1-yl, 2-carbamido-2,2-dimethyleth-1-yl, 2- or 3-carbamidoprop-1-yl, 2-, 3- or 4-carbamidobut-1-yl, 3-carbamido-2-methylprop-1-yl, 3-carbamido-1,2-dimethylprop-1-yl, 3-carbamido-3-methylprop-1-yl, 3-carbamido-2,2-dimethylprop-1-yl, 2-, 3-, 4- or 5-carbamidopent-1-yl, 4-carbamido-3,3- or -2,2-dimethylbut-1-yl.

As $C_1$-$C_6$-alkyl-HN-C(O)-$C_1$-$C_6$-alkyl or $(C_1$-$C_6$-alkyl$)_2$N-C(O)-$C_1$-$C_6$-alkyl, $R^5$ may be linear or branched, and the NH-alkyl group contains preferably from 1 to 4 carbon atoms, and the alkyl group preferably from 2 to 6 carbon atoms. Examples are the aforementioned carbamidoalkyl groups whose nitrogen atom is substituted by one or two methyl, ethyl, propyl or butyl.

Halogen means, for example, F, Cl, Br or I, preferably F or Cl.

Examples of $C_1$-$C_6$-alkyl and -alkoxy radicals are methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy respectively.

$C_1$-$C_6$-Alkylenedioxy radicals are preferably methylenedioxy, ethylenedioxy and propylenedioxy. Examples of $C_1$-$C_6$-alkanoyl radicals are acetyl, propionyl and butyryl.

Cycloalkyl means a saturated, cyclic hydrocarbon radical having 3-8 carbon atoms, for example cyclopropyl, cyclobutyl, cyclopentyl and cyclohexyl.

$C_1$-$C_6$-Alkylene radicals are, for example, methylene, ethylene, propylene, 2-methylpropylene, tetra-, penta- and hexamethylene; $C_2$-$C_6$-alkenylene radicals are, for example, vinylene and propenylene; $C_2$-$C_6$-alkynylene radicals are, for example, ethynylene; acyl radicals are alkanoyl radicals, preferably $C_1$-$C_6$-alkanoyl radicals, or aroyl radicals such as benzoyl. Aryl denotes mono- or polycyclic aromatic radicals which may be mono- or polysubstituted, for example phenyl, substituted phenyl, naphthyl, substituted naphthyl, tetrahydronaphthyl or substituted tetrahydronaphthyl.

Examples of substituents on aryl radicals, heterocyclyl radicals and polycyclic, unsaturated hydrocarbon radicals are $C_1$-$C_6$-alkyl, $C_{3-8}$-cycloalkyl, $C_{3-8}$-cydoalkoxy, $C_{3-8}$-cydoalkoxy-$C_{1-6}$-alkyl, $C_{3-8}$-cydoalkoxy-$C_{1-6}$-alkoxy, $C_1$-$C_6$-alkylamino, di-$C_1$-$C_6$-alkylamino, amino-$C_{1-6}$-alkyl, amino-$C_{2-7}$-alkoxy, polyhalo-$C_{1-6}$-alkyl, in particular trifluoromethyl, polyhalo-$C_{2-7}$-alkoxy, nitro, amino, $C_2$-$C_6$-alkenyl, $C_2$-$C_6$-alkynyl, $C_1$-$C_6$-alkoxy, $C_1$-$C_6$-alkanoyloxy, hydroxy, halogen, oxo, oxide, cyano, carbamoyl, carboxyl, $C_1$-$C_6$-alkylenedioxy, phenyl, phenoxy, phenylthio, phenyl-$C_1$-$C_6$-alkyl, phenyl- $C_1$-$C_6$-alkoxy, $C_{1-6}$-alkoxycarbonylphenyl, hydroxy-$C_{1-6}$-alkylphenyl, benzyloxy, pyridylcarbonylamino-$C_{1-6}$-alkyl, $C_{2-6}$-alkenyloxy, $C_{1-6}$-alkoxy-$C_{1-6}$-alkoxy, $C_{1-6}$-alkoxy-$C_{1-6}$-alkoxy-$C_{1-6}$-alkyl, methoxybenyloxy, hydroxybenzyloxy, phenethyloxy, methylenedioxybenzyloxy, dioxolanyl-$C_{1-6}$-alkoxy, cyclopropyl-$C_{1-6}$-alkyl, cydopropyl-$C_{1-6}$-alkoxy, hydroxy-$C_{1-6}$-alkoxy, carbamoyloxy-$C_{1-6}$-alkoxy, pyridylcarbamoyloxy-$C_{1-6}$-alkoxy, benzoyloxy-$C_{1-6}$-alkoxy, $C_{1-6}$-alkoxycarbonyl, $C_{1-6}$-alkylcarbonylamino, $C_{1-6}$-alkylcarbonylamino-$C_{1-6}$-alkyl, $C_{1-6}$-alkylcarbonylamino-$C_{2-7}$-alkoxy, (N-$C_{1-6}$-alkyl)-$C_{1-6}$-alkyl-carbonylamino-$C_{1-6}$-alkyl, (N-$C_{1-6}$-alkyl)-$C_{1-6}$-alkylcarbonylamino-$C_{2-7}$-alkoxy, $C_{3-8}$-cycloalkyl-carbonylamino-$C_{1-6}$-alkyl, $C_{3-8}$-cycloalkylcarbonylamino-$C_{2-7}$-alkoxy, $C_{1-6}$-alkoxy-$C_{1-6}$-alkyl, hydroxy-$C_{1-6}$-alkyl, hydroxy-$C_{2-7}$-alkoxy-$C_{1-6}$-alkyl, hydroxy-$C_{2-7}$-alkoxy-$C_{1-6}$-alkoxy, $C_{1-6}$-alkoxycarbonylamino-$C_{1-6}$-alkyl, $C_{1-6}$-alkoxycarbonylamino-$C_{2-7}$-alkoxy, $C_{1-6}$-alkylamino-carbonylamino-$C_{1-6}$-alkyl, $C_{1-6}$-alkylaminocarbonylamino-$C_{2-7}$-alkoxy, $C_{1-6}$-alkylaminocarbonyl-$C_{1-6}$-alkyl, $C_{1-6}$-alkylaminocarbonyl-$C_{1-6}$-alkoxy, $C_{1-6}$-alkylaminocarbonyl-$C_{1-6}$-alkoxy-$C_{1-6}$-alkyl, di-$C_{1-6}$-alkylaminocarbonyl-$C_{1-6}$-alkyl, di-$C_{1-6}$-alkylaminocarbonyl-$C_{1-6}$-alkoxy, $C_{1-6}$-alkyl-carbonyloxy-$C_{1-6}$-alkyl, $C_{1-6}$-alkylcarbonyloxy-$C_{1-6}$-alkoxy, cyano-$C_{1-6}$-alkyl, cyano-$C_{1-6}$-alkoxy, 2-oxooxazolidinyl-$C_{1-6}$-alkyl, 2-oxooxazolidinyl-$C_{1-6}$-alkoxy, $C_{1-6}$-alkoxycarbonyl-$C_{1-6}$-alkyl, $C_{1-6}$-alkoxycarbonyl-$C_{1-6}$-alkoxy, $C_{1-6}$-alkylsulphonylamino-$C_{1-6}$-alkyl, $C_{1-6}$-alkylsulphonylamino-$C_{2-7}$-alkoxy, (N-$C_{1-6}$-alkyl)-$C_{1-6}$-alkylsulphonylamino-$C_{1-6}$-alkyl, (N-$C_{1-6}$-alkyl)-$C_{1-6}$-alkylsulphonylamino-$C_{2-7}$-alkoxy, $C_{1-6}$-alkylamino-$C_{1-6}$-alkyl, $C_{1-6}$-alkylamino-$C_{2-7}$-alkoxy, di-$C_{1-6}$-alkylamino-$C_{1-6}$-alkyl, di-$C_{1-6}$-alkylamino-$C_{2-7}$-alkoxy, $C_{1-6}$-alkylsulphonyl-$C_{1-6}$-alkyl, $C_{1-6}$-alkylsulphonyl-$C_{1-6}$-alkoxy, carboxy-$C_{1-6}$-alkyl, carboxy-$C_{1-6}$-alkoxy, carboxy-$C_{1-6}$-alkoxy-$C_{1-6}$-alkyl, $C_{1-6}$-alkoxy-$C_{1-6}$-alkylcarbonyl,

acyl-$C_{1-6}$-alkoxy-$C_{1-6}$-alkyl, (N-$C_{1-6}$-alkyl)-$C_{1-6}$-alkoxy-carbonylamino, (N-hydroxy)-$C_{1-6}$-alkylaminocarbonyl-$C_{1-6}$-alkyl, (N-hydroxy)-$C_{1-6}$-alkylaminocarbonyl-$C_{1-6}$-alkoxy, (N-hydroxy)aminocarbonyl-$C_{1-6}$-alkyl, (N-hydroxy)aminocarbonyl-$C_{1-6}$-alkoxy,$C_{1-6}$-alkoxyaminocarbonyl-$C_{1-6}$-alkyl,6-alkoxyaminocarbonyl-$C_{1-6}$-alkoxy,(N-$C_{1-6}$-alkoxy)-$C_{1-6}$-alkylaminocarbonyl-$C_{1-6}$-alkyl, (N-$C_{1-6}$-alkoxy)-$C_{1-6}$-alkylaminocarbonyl-$C_{1-6}$-alkoxy, (N-acyl)-$C_{1-6}$-alkoxy-$C_{1-6}$-alkylamino, $C_{1-6}$-alkoxy-$C_{1-6}$-alkylcarbamoyl, (N-$C_{1-6}$-alkyl)-$C_{1-6}$-alkoxy-$C_{1-6}$-alkylcarbamoyl, $C_{1-6}$-alkoxy-$C_{1-6}$-alkylcarbonyl, $C_{1-6}$-alkoxy-$C_{1-6}$-alkylcarbonylamino, (N-$C_{1-6}$-alkyl)-$C_{1-6}$-alkoxy-$C_{1-6}$-alkylcarbonylamino, 1-$C_{1-6}$-alkoxy-$C_{1-6}$-alkylimidazol-2-yl, 1-$C_{1-6}$-alkoxy-$C_{1-6}$-alkyltetrazol-5-yl, 5-$C_{1-6}$-alkoxy-$C_{1-6}$-alkyltetrazol-1-yl, 2-$C_{1-6}$-alkoxy-$C_{1-6}$-alkyl-4-oxo-imidazol-1-yl, carbamoyl-$C_{1-6}$-alkyl, carbamoyl-$C_{1-6}$-alkoxy, $C_{1-6}$-alkylcarbamoyl, di-$C_{1-6}$-alkylcarbamoyl, $C_{1-6}$-alkylsulphonyl, $C_{1-6}$-alkylamidinyl, acetamidinyl-$C_{1-6}$-alkyl, O-methyl-oximyl-$C_{1-6}$-alkyl, O,N-dimethylhydroxylamino-$C_{1-6}$-alkyl, $C_{3-6}$-cydoalkyl-$C_{1-6}$-alkanoyl, aryl-$C_{1-6}$-alkanoyl or heterocyclyl-$C_{1-6}$-alkanoyl, each of which is optionally substituted by halogen, $C_1$-$C_6$-alkyl, $C_{1-6}$-alkoxy, hydroxyl, $C_1$-$C_6$-alkylamino, di- $C_1$-$C_6$-alkylamino, $C_{1-6}$-alkoxycarbonyl, hydroxy-$C_{1-6}$-alkyl or trifluoromethyl; and pyridyl, pyridyloxy, pyridylthio, pyridylamino, pyridyl-$C_{1-6}$-alkyl, pyridyl-$C_{1-6}$-alkoxy, pyrimidinyl, pyrimidinyloxy, pyrimidinylthio, pyrimidinylamino, pyrimidinyl-$C_{1-6}$-alkyl, pyrimidinyl-$C_{1-6}$-alkoxy, thienyl, thienyl-$C_{1-6}$-alkyl, thienyl-$C_{1-6}$-alkoxy, furyl, furyl-$C_{1-6}$-alkyl or furyl-$C_{1-6}$-alkoxy, each of which is optionally substituted by halogen, $C_{1-6}$-alkyl, $C_{1-6}$-alkoxyl or dihydroxy-$C_{1-6}$-alkylaminocarbonyl.

The term polycyclic, unsaturated hydrocarbon radical comprises aromatic radicals and denotes radicals such as naphthyl, cyclohexenophenyl, indanyl and acenaphthyl, for example.

The term heterocyclyl denotes mono- or bicyclic, saturated. unsaturated and aromatic heterocyclic radicals having from 1 to 4 nitrogen and/or 1 or 2 sulphur or oxygen atoms, each of which may be mono- or polysubstituted, in particular mono-, di- or trisubstituted. In addition, the term heterocyclyl encompasses the above oxo-substituted radicals. Examples of heterocyclyl radicals are azepanyl, aziridinyl, dioxolanyl, dioxanyl, dithiolanyl, dithianyl, oxepanyl, pyrrolidinyl, piperidinyl, piperazinyl, pyridyl, thiepanyl, thienyl, pyrazinyl, triazolyl, imidazolyl, benzthiazolyl, furyl, pyranyl, tetrahydrofuranyl, tetrahydropyranyl, tetrahydrothiophenyl, tetrahydrothiopyranyl, azetidinyl, pyrimidinyl, morpholinyl, thiomorpholinyl, quinazolinyl, quinolyl, quinoxalinyl, isoquinolyl, benzo[b]thienyl, isobenzofuranyl, benzoimidazolyl, oxazolyl, thiazolyl, indolyl, pyrrolyl, pyrazolyl, triazinyl, dihydrobenzofuranyl, tetrahydroquinoxalinyl, dihydro-3H-benzo[1,4]oxazinyl, 1H-pyrrolizinyl, pthalazinyl, [1,5]naphthyridyl, dihydro-2H-benzo[1,4]thiazinyl, dihydro-1H-pyrido[2,3-b][1,4]oxazinyl, 1 H-pyrrolo[2,3-b]pyridyl, benzo-[1,3]dioxolyl, benzooxazolyl, 2,3-dihydroindolyl, indazolyl or benzofuranyl. Examples of substituted heterocyclyl radicals are 1-methylpiperidinyl, 1-methylpyrrolidinyl, 4-methylpiperazinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopiperidinyl, 2-oxopyrrolidinyl, 2-oxo[1,3]oxazinyl, 2-oxobenzimidazolyl, 2-oxodihydro-benzo[d][1,3]oxazinyl, 4-oxodihydroimidazolyl, 5-oxo-4H-[1,2,4]triazinyl, 3-oxo-4H-benzo-[1,4]thiazinyl, 1,1,3-trioxodihydro-2H-1$\lambda^6$-benzo[1,4]thiazinyl, 1-oxopyridyl, 2-oxotetra-hydrobenzo[e][1,4]diazepinyl, 2-oxodihydrobenzo[e][1,4]diazepinyl, 1-oxo-3H-isobenzofuranyl, 4-oxo-3H-thieno[2,3-d]pyrimidinyl, 3-oxo-4H-benzo[1,4]oxazinyl, 1,1-dioxodihydro-2H-benzo[1,4]thiazinyl, 2-oxo-1H-pyrido[2,3-b][1,4]oxazinyl, 2-oxobenzooxazolyl, 2-oxo-1,3-dihydroindolyl, 1-methyl-1 H-indazolyl, 3-methyl-1 H-indazolyl, 3-methyl-1 H-indolyl, 1-methyl-1 H-indolyl, methoxypyridyl, 2-oxoazepanyl or 2-oxotetrahydropyrimidinyl.

In the case of $R^1$ and $R^5$, the heterocyclyl radicals may additionally also be substituted by heterocyclylalkyl, heterocyclylalkoxy, heterocyclylalkoxyalkyl or heterocyclyl, for example piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-ylalkoxy, 3-methyl-[1,2,4]oxadiazol-5-ylalkyl, 3-methyl[1,2,4]oxadiazol-5-ylalkoxy, 5-methyl[1,2,4]oxadiazol-3-ylalkyl, 5-methyl[1,2,4]oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-$C_{1-6}$-alkoxy-$C_{1-6}$-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl, 2-oxotetrahydropyrimidinyl or by the -O-$CH_2CH(OH)CH_2NR_x$ radical where $NR_x$ is a mono- or di-$C_{1-6}$-alkylamino, piperidino, morpholino, piperazino or N-methylpiperazino radical.

The term polyhydroxyalkyl denotes $C_1$-$C_7$-alkyl radicals which may be substituted by 2-6 hydroxyl groups, for example glyceryl, arabityl, sorbityl, etc.

The compounds of the formula (I) have at least four asymmetric carbon atoms and can therefore be present in the form of optically pure diastereomers, diastereomer mixtures, diastereomeric racemates, mixtures of diastereomeric racemates or as a meso compounds. The invention encompasses all of these forms. Diastereomer mixtures, diastereomeric racemates or mixtures of diastereomeric racemates may be separated by customary methods, for example by column chromatography, thin-layer chromatography, HPLC and the like.

Salts of compounds having salt-forming groups are in particular acid addition salts, salts with bases or, in the presence of a plurality of salt-forming groups, in some cases also mixed salts or internal salts.

Salts are primarily the pharmaceutically usable or nontoxic salts of compounds of the formula (I).

Such salts are formed, for example, from compounds of the formula (I) with an acidic group, for example a carboxyl or sulpho group, and are, for example, the salts thereof with suitable bases, such as nontoxic metal salts derived from metals of group Ia, Ib, IIa and IIb of the Periodic Table of the Elements, for example alkali metal, in particular lithium, sodium or potassium salts, alkaline earth metal salts, for example magnesium or calcium salts, and also zinc salts or ammonium salts, including those salts which are formed with organic amines, such as optionally hydroxy-substituted mono-, di- or trialkylamines, in particular mono-, di- or tri(lower alkyl)amines, or with quaternary ammonium bases, for example methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amines, such as N,N-dimethyl-N-(2-hydroxyethyl)amine, or N-methyl-D-glucamine, or quaternary ammonium hydroxides, such as tetrabutylammonium hydroxide. The compounds of the formula I having a basic group, for example an amino group, may form acid addition salts, for example with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic, sulpho or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the α-amino acids mentioned above, and also methanesulphonic acid, ethanesulphonic acid, 2-hydroxyethanesulphonic acid, ethane-1,2-disulphonic acid, benzenesulphonic acid, 4-methylbenzenesulphonic acid, naphthalene-2-sulphonic acid, 2- or 3-phosphoglycerate, glucose-6-phosphate, N-cyclohexylsulphamic acid (with formation of cyclamates) or with other acidic organic compounds such as ascorbic acid. Compounds of the formula (I) with acidic and basic groups may also form internal salts.

For the isolation and purification, pharmaceutically unsuitable salts may also find use.

The compound groups mentioned below are not to be regarded as closed, but rather parts of these compound groups may be exchanged with one another or with the definitions given above or omitted in a sensible manner, for example to replace general by more specific definitions.

Preferred inventive compounds are those of the general formula (IA)

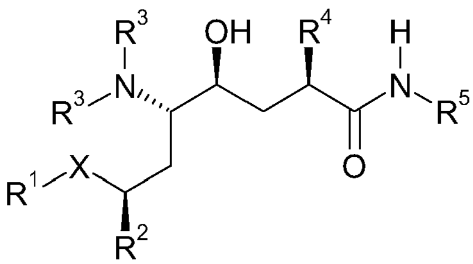

(IA)

or a pharmaceutically usable salt thereof; wherein $R^1$, $R^2$, $R^3$, $R^4$, $R^5$ and X are each as defined above for the compounds of the formula (I).

Further preferred groups of compounds of the formula (I) or a pharmaceutically usable salt thereof, or more preferably of the formula (IA) or a pharmaceutically usable salt thereof, are compounds in which at least one, and most preferred all, substituent(s) is (are) defined as follows:

X is $CH_2$;

$R^1$ is as specified for (A) or (B), preferably as specified for (A);

$R^2$ is $C_1$-$C_6$-alkyl;

$R^3$ is H;

$R^4$ is $C_1$-$C_6$-alkyl; and

$R^5$ is $C_1$-$C_6$-alkyl, $C_1$-$C_6$-alkoxy-$C_1$-$C_6$-alkyl, halo-$C_1$-$C_6$-alkyl, $C_2$-$C_8$-alkynyl, cyano-$C_1$-$C_6$-alkyl, optionally substituted $C_3$-$C_6$-cycloalkyl, $C_3$-$C_8$-cycloalkyl-$C_1$-$C_6$-alkyl, optionally substituted aryl, optionally substituted heterocyclyl-$C_0$-$C_6$-alkyl which, for $C_0$-alkyl, is bonded via a carbon atom or $H_2N$-C(O)-$C_1$-$C_6$-alkyl.

Especially preferred $R^1$ radicals are benzoimidazolyl, di-$C_{1-6}$-alkoxypyrimidinyl, 2- or 5-benzo[b]thienyl, 6- or 7-isoquinolyl, 6- or 7-tetrahydroquinolyl, 6- or 7-tetrahydroisoquinolyl, 6-quinoxalinyl, 6- or 7-quinazolinyl, dihydro-3H-benzo[1,4]oxazinyl, 3,4-dihydro-2H-benzo[1,4]oxazinyl, 3-oxo-4H-benzo[1,4]oxazinyl, 2-oxobenzooxazolyl, 2-oxo-1,3-dihydroindolyl, 2,3-dihydroindolyl, indazolyl, benzofuranyl, 6- or 7-quinolyl, 6- or 7-isoquinolyl, 6- or 7-tetrahydroquinolyl, oxotetrahydroquinolyl, 6- or 7-tetrahydroisoquinolyl, 6-quinoxalinyl, 6- or 7-quinazolinyl, indolyl, 3-oxo-3,4-dihydro-2H-benzo[1,4]oxazinyl, 2-oxo-2,3-dihydro-benzooxazolyl, 2,3-dihydrobenzothiazinyl, imidazolyl, pyridinyl, pyrrolo[2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,5-a]pyridinyl, imidazo[1,2-a]pyrimidinyl, naphthyl and cyclohexenophenyl, each of which is substituted by from one to four radicals selected from hydroxy, halogen, oxo, oxide, carbamoyl, carboxyl, cyano, trifluoromethyl, $C_{1-6}$-alkyl, $C_{1-6}$-alkoxy, hydroxy-$C_{1-6}$-alkoxy, $C_{1-6}$-alkoxy-$C_{1-6}$-alkyl, $C_{1-6}$-alkoxy-$C_{1-6}$-alkoxy, di-$C_{1-6}$-alkylamino, 2,3-dihydroxypropoxy, 2,3-dihydroxypropoxy-$C_{1-6}$-alkoxy, 2,3-dimethoxypropoxy, methoxybenzyloxy, hydroxybenzyloxy, phenethyloxy, methylenedioxybenzyloxy, dioxolanyl-$C_{1-6}$-alkoxy, cyclopropyl-$C_{1-6}$-alkoxy, pyridylcarbamoyloxy-$C_{1-6}$-alkoxy, 3-morpholino-2-hydroxypropoxy, benzyloxy-$C_{1-6}$-alkoxy, picolyloxy, $C_{1-6}$-alkoxycarbonyl, $C_{1-6}$-alkoxy-$C_{1-6}$-alkoxy-$C_{1-6}$-alkyl, $C_{1-6}$-alkylcarbonylamino, $C_{1-6}$-alkylcarbonylamino-$C_{1-6}$-alkyl, $C_{1-6}$-alkylcarbonylamino-$C_{1-6}$-alkoxy, (N-$C_{1-6}$-alkyl)-$C_{1-6}$-alkylcarbonylamino-$C_{1-6}$-alkyl, (N-$C_{1-6}$-alkyl)-$C_{1-6}$-alkylcarbonylamino-$C_{1-6}$-alkoxy, $C_{3-6}$-cycloalkylcarbonylamino-$C_{1-6}$-alkyl, $C_{3-6}$-cycloalkyl-carbonylamino-$C_{1-6}$-alkoxy, $C_{1-6}$-alkoxy-$C_{1-6}$-alkyl, hydroxy-$C_{1-6}$-alkyl, hydroxy-$C_{1-6}$-alkoxy-$C_{1-6}$-alkyl, hydroxy-$C_{1-6}$-alkoxy-$C_{1-6}$-alkoxy, $C_{1-6}$-alkoxycarbonylamino-$C_{1-6}$-alkyl, $C_{1-6}$-alkoxycarbonylamino-$C_{1-6}$-alkoxy, $C_{1-6}$-alkylaminocarbonylamino-$C_{1-6}$-alkyl, $C_{1-6}$-alkylamino-carbonylamino-$C_{1-6}$-alkoxy, $C_{1-6}$-alkylaminocarbonyl-$C_{1-6}$-alkyl, $C_{1-6}$-alkylaminocarbonyl-$C_{1-6}$-alkoxy, $C_{1-6}$-alkylaminocarbonyl-$C_{1-6}$-alkoxy-$C_{1-6}$-alkyl, di-$C_{1-6}$-alkylaminocarbonyl-$C_{1-6}$-alkyl, di-$C_{1-6}$-alkylaminocarbonyl-$C_{1-6}$-alkoxy, $C_{1-6}$-alkylcarbonyloxy-$C_{1-6}$-alkyl, $C_{1-6}$-alkyl-carbonyloxy-$C_{1-6}$-alkoxy, cyano-$C_{1-6}$-alkyl, cyano-$C_{1-6}$-alkoxy, 2-oxooxazolidinyl-$C_{1-6}$-alkyl, 2-oxooxazolidinyl-$C_{1-6}$-alkoxy, $C_{1-6}$-alkoxycarbonyl-$C_{1-6}$-alkyl, $C_{1-6}$-alkoxycarbonyl-$C_{1-6}$-alkoxy, $C_{1-6}$-alkylsulphonylamino-$C_{1-6}$-alkyl, $C_{1-6}$-alkylsulphonylamino-$C_{1-6}$-alkoxy, (N-$C_{1-6}$-alkyl)-$C_{1-6}$-alkylsulphonylamino-$C_{1-6}$-alkyl, (N-$C_{1-6}$-alkyl)-$C_{1-6}$-alkylsulphonylamino-$C_{1-6}$-alkoxy, $C_{1-6}$-alkylamino-$C_{1-6}$-alkyl, $C_{1-6}$-alkylamino-$C_{1-6}$-alkoxy, di-$C_{1-6}$-alkylamino-$C_{1-6}$-alkyl, di-$C_{1-6}$-alkylamino-$C_{1-6}$-alkoxy, $C_{1-6}$-alkylsulphonyl-$C_{1-6}$-alkyl, $C_{1-6}$-alkylsulphonyl-$C_{1-6}$-alkoxy, carboxy-$C_{1-6}$-alkyl, carboxy-$C_{1-6}$-alkoxy, carboxy-$C_{1-6}$-alkoxy-$C_{1-6}$-alkyl, $C_{1-6}$-alkoxy-$C_{1-6}$-alkylcarbonyl, acyl-$C_{1-6}$-alkoxy-$C_{1-6}$-alkyl, (N-$C_{1-6}$-alkyl)-$C_{1-6}$-alkoxycarbonylamino, (N-hydroxy)-$C_{1-6}$-alkylaminocarbonyl-$C_{1-6}$-alkyl, (N-hydroxy)-$C_{1-6}$-alkylaminocarbonyl-$C_{1-6}$-alkoxy, (N-hydroxy)aminocarbonyl-$C_{1-6}$-alkyl, (N-hydroxy)aminocarbonyl-$C_{1-6}$-alkoxy,$C_{1-6}$-alkoxy-aminocarbonyl-$C_{1-6}$-alkyl,6-alkoxyaminocarbonyl-$C_{1-6}$-alkoxy,(N-$C_{1-6}$-alkoxy)-$C_{1-6}$-alkylaminocarbonyl-$C_{1-6}$-alkyl, (N-$C_{1-6}$-alkoxy)-$C_{1-6}$-alkylaminocarbonyl-$C_{1-6}$-alkoxy, (N-acyl)-$C_{1-6}$-alkoxy-$C_{1-6}$-alkylamino, $C_{1-6}$-alkoxy-$C_{1-6}$-alkylcarbamoyl, (N-$C_{1-6}$-alkyl)-$C_{1-6}$-alkoxy-$C_{1-6}$-alkylcarbamoyl, $C_{1-6}$-alkoxy-$C_{1-6}$-alkylcarbonyl, $C_{1-6}$-alkoxy-$C_{1-6}$-alkylcarbonylamino, (N-$C_{1-6}$-alkyl)-$C_{1-6}$-alkoxy-$C_{1-6}$-alkylcarbonylamino, 1-$C_{1-6}$-alkoxy-$C_{1-6}$-alkylimidazol-2-yl, 1-$C_{1-6}$-alkoxy-$C_{1-6}$-alkyltetrazol-5-yl, 5-$C_{1-6}$-alkoxy-$C_{1-6}$-alkyltetrazol-1-yl, 2-$C_{1-6}$-alkoxy-$C_{1-6}$-alkyl-4-oxoimidazol-1-yl, carbamoyl-$C_{1-6}$-alkyl, carbamoyl-$C_{1-6}$-alkoxy, $C_{1-6}$-alkylcarbamoyl, di-$C_{1-6}$-alkylcarbamoyl, $C_{1-6}$-alkylsulphonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-ylalkoxy, 3-methyl[1,2,4]oxadiazol-5-ylalkyl, 3-methyl[1,2,4]oxadiazol-5-yl-alkoxy, 5-methyl[1,2,4]oxadiazol-3-ylalkyl, 5-methyl[1,2,4]oxadiazol-3-ylalkoxy, tetrazol-1-yl-alkyl, tetrazol-1-ylalkoxy, tetrazol-2-ylalkyl, tetrazol-2-ylalkoxy, tetrazol-5-ylalkyl, tetrazol-5-yl-alkoxy, 5-methyltetrazol-1-ylalkyl, 5-methyltetrazol-1-ylalkoxy, thiazol-4-ylalkyl, thiazol-4-yl-alkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-$C_{1-6}$-alkoxy-$C_{1-6}$-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl and 2-oxotetrahydropyrimidinyl, where, in the case of naphthyl, or cyclohexenophenyl, at least the ring system not bonded to X is substituted as specified.

Further especially preferred $R^1$ radicals are heterocyclic radicals, in particular benzoimidazolyl, 3,4-dihydro-2H-benzo[1,4]oxazinyl, indazolyl, benzofuranyl, indolyl, pyridinyl, pyrrolo[2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,5-a]pyridinyl and imidazo[1,2-a]pyrimidinyl, each of which is substituted by from one to four radicals selected from hydroxy, halogen, oxo, oxide, carbamoyl, carboxyl, cyano, trifluoromethyl, $C_{1-6}$-alkyl, $C_{1-6}$-alkoxy, hydroxy-$C_{1-6}$-alkoxy,

$C_{1-6}$-alkoxy-$C_{1-6}$-alkyl and $C_{1-6}$-alkoxy-$C_{1-6}$-alkoxy.

The compounds of the formula (I) or a pharmaceutically usable salt thereof may be prepared in an analogous manner to the preparation processes known from the literature. Similar preparation processes are described, for example, in EP 678503, WO 01/09079, WO 01/09083, WO 02/02487, WO 02/02500, WO 02/02508, WO 02/08172, WO 02/092828 and in Helvetica Chemica Acta 86 (2003), 2848-2870 and literature cited there (scheme).

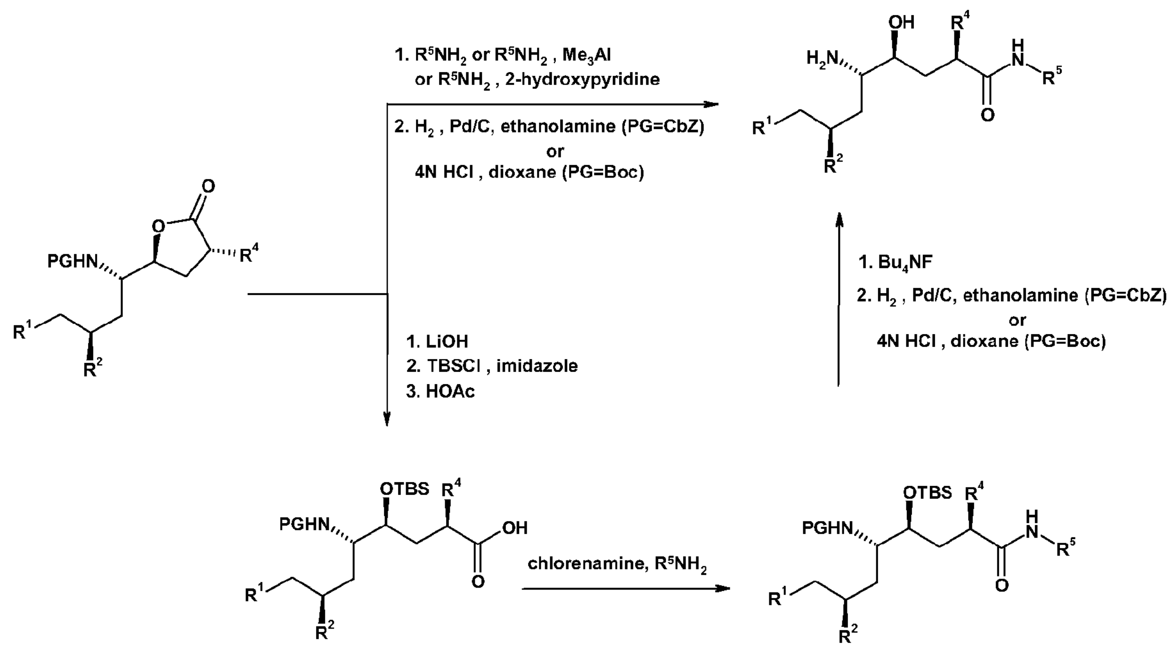


Details of the specific preparation variants can be taken from the examples.

The compounds of the formula (I) or a pharmaceutically usable salt thereof may also be prepared in optically pure form. The separation into antipodes may be effected by methods known per se, either preferably at a synthetically early stage by salt formation with an optically active acid, for example (+)- or (-)-mandelic acid and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the piperidine present, the pure diastereomeric salts and derivatives may be analysed with common spectroscopic methods, of which X-ray spectroscopy on single crystals constitutes a particularly suitable method.

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, and the acyl group is as defined in the present context. Preference is given to pharmaceutically usable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower $\omega$-(amino, mono- or dialkylamino, carboxyl, lower alkoxycarbonyl)-alkyl esters or such as lower $\alpha$-(alkanoyloxy, alkoxycarbonyl or dialkylaminocarbonyl)-alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where this is possible and appropriate.

The compounds of the formula (I) or a pharmaceutically usable salt thereof also include those compounds in which one or more atoms are replaced by their stable, non-radioactive isotopes; for example, a hydrogen atom by deuterium.

The compounds of formula (I) and (IA), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance

energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 ul buffer, 10 ul inhibitor in DMSO, 10 ul peptide substrate in DMSO and 20 ul enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 μl of a 1.0 M Tris Base solution. The enzymatic substrate to product conversion is assessed by fluorescence measurements at 460 nm wave length.

In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (IA), respectively, and their pharmaceutically useful salts for the prevention, delay of progression or the treatment of Alzheimer Disease, malaria or HIV infection.

Subject of the present invention is also the use of the compounds of formula (I) and (IA), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or a pharmaceutically usable salt thereof or preferred formula (IA) or a pharmaceutically usable salt thereof is applied.

Subject of the present invention is also a pharmaceutical preparation that contains for the inhibition of beta-secretase, cathepsin D, plasmepsin and/or HIV-protease a compound of the general formula (I) or a pharmaceutically usable salt thereof, or preferred of formula (IA) or a pharmaceutically usable salt thereof as well as commonly used ingredients.

Subject of the present invention is also a pharmaceutical preparation for the prevention, delay of progression or treatment of Alzheimer Disease, malaria and HIV infection that contains a compound of the general formula (I) or a pharmaceutically usable salt thereof, or preferred of formula (IA) or a pharmaceutically usable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The examples which follow are intended to illustrate the present invention, but not to restrict it in any way. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature). Unless stated otherwise, the absolute stereochemistry of the "main chain substituents" is (2S,4S,5S,7S) (see formula II).

The Example Compounds 1A to 1YY correspond to the formula (II)

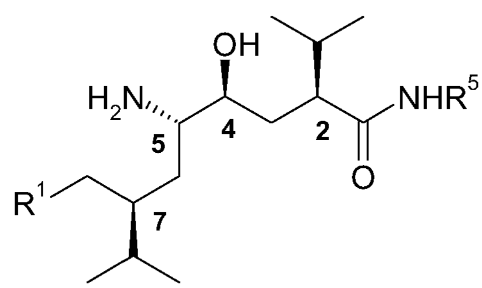

(II)

,

wherein $R^1$ and $NHR^5$ correspond to the below-specified residues, which, unless stated otherwise below are prepared as described in detail in WO2005/090305 pages 20 to 39 and 39 to 41, respectively, which description is herewith incorporated. R 1 and $NHR^5$ in each example compound 1A to 1YY corresponds to one of the below-specified residues A to YY. The atoms denoted by * are the bonding sites. The further example compounds 2A to 40YY are accordingly the compounds of formula (II) in which the $NHR^5$ radical assumes all above residue-definitions (A to YY) for a given $R^1$ (above residue-definitions 2 to 40). Thus, example compound 1K is the compound N-(2-carbamoyl-2-methylpropyl)-5-amino-4-hydroxy-2-isopropyl-7-[3-(3-methoxypropyl)-1H-indol-5-ylmethyl]-8-methylnonanamide.

Analogously to that preparation processes, the remaining compounds 1A to 40YY are obtained.

Details of specific preparation variants can be taken from the examples.

HPLC gradients on Hypersil BDS C-18 (5 μm); column: 4 x 125 mm

| | |
|---|---|
| I | 90% water*/10% acetonitrile* to 0% water*/100% acetonitrile* in 5 minutes + 2.5 minutes (1.5 ml/min) |

II 95% water*/5% acetonitrile* to 0% water*/100% acetonitrile* in 40 minutes (0.8 ml/min)

* contains 0.1 % trifluoroacetic acid

The following abbreviations are used:

Rf ratio of distance travelled by a substance to separation of the eluent front from the start point in thin-layer chromatography

Rt retention time of a substance in HPLC (in minutes)

m.p. melting point (temperature)

b.p. boiling point (temperature)

General method A: (N-CbZ deprotection)

A solution of 1 mmol of "N-CbZ derivative" in 30 ml of ethanol is hydrogenated in the presence of 0.1 mmol of ethanolamine and 0.150 g of 10% Pd/C at 0-10°C over 1-3 hours. The reaction mixture is clarified by filtration and the filtrate is concentrated by evaporation. The residue is admixed with 30 ml of 1M sodium hydrogen carbonate solution and extracted with tert-butyl methyl ether (2x). The combined organic phases are dried over sodium sulphate and filtered, and the filtrate is concentrated by evaporation. The title compound is obtained from the residue by means of flash chromatography ($SiO_2$ 60F).

General method B: (N-Boc-deprotection))

A solution of 1 mmol of "amine" in 20 ml of dichloromethane at 0°C is treated with 50 mmol of trifluoroacetic acid. The reaction mixture stirred for 1-3 hours at 0°C, neutralized with 1M sodium bicarbonate solution and extracted with tert-butyl methyl ether (3x). The combined organic phases are washed with brine, dried over sodium sulphate and filtered, and the filtrate is concentrated by evaporation. The title compound is obtained from the residue by means of flash chromatography ($SiO_2$ 60F).

General method C: (lactone amidation I)

A mixture of 1 mmol of "lactone", "amine" (5-30 equiv.) (methylamine/ethylamine are used as a 10% solution in triethylamine) and 2-hydroxypyridine (1.0 equiv.) is stirred at 40-50°C over 2-16 hours. The reaction mixture is admixed with 30 ml of 1M sodium bicarbonate solution and extracted with tert-butyl methyl ether (2x). The combined organic phases are dried over sodium sulphate and filtered, and the filtrate is concentrated by evaporation. The title compound is obtained from the residue by means of flash chromatography ($SiO_2$ 60F).

General method D: (lactone amidation II)

A solution of 1.1 mmol of trimethylaluminium solution (2M in heptane) at -78°C is admixed with a solution of 1.2 mmol of "amine" in 1-2 ml of toluene. The reaction mixture is warmed to room temperature, stirred further for 30-60 minutes and subsequently concentrated by evaporation. The residue is admixed with a solution of 1 mmol of "lactone" in 2 ml of toluene and stirred at 80°C over 2-4 hours. The reaction mixture is cooled to room temperature, admixed with 10 ml of 1 N HCl and then stirred for a further 30 minutes. The reaction mixture is diluted with brine and extracted with toluene (2x) - the combined organic phases are dried over sodium sulphate and concentrated by evaporation. The title compound is obtained from the residue by means of flash chromatography ($SiO_2$ 60F).

General method E: (chlorenamine-coupling/desilylation)

A solution of 1 mmol of "acid" in 10 ml of dichloromethane at 0°C is treated with 1.2-1.8 mmol of (1-chloro-2-methyl-propenyl)-dimethyl-amine (chlorenamine). After stirring for 1-4 hours, the reaction mixture is concentrated by evaporation and the residue is dissolved in 6 ml of dichloromethane. The solution is added dropwise over 2-10 minutes

to a solution of 1.25 mmol of "amine" and 1.1 mmol of triethylamine in 6 ml of dichloromethane at 0˚C. The reaction mixture stirred for 1-16 hours at room temperature, poured onto water and extracted with tert-butyl methyl ether (2x). The combined organic phases are washed with brine, dried over sodium sulphate and filtered, and the filtrate is concentrated by evaporation. The residue is dissolved in 10-15 ml of tetrahydrofuran at 0˚C and treated with 1.5 mmol of tetrabutylammonium fluoride solution (1M in tetrahydrofuran). The reaction mixture stirred for 2-4 hours at room temperature, poured onto 1M sodium bicarbonate solution and extracted with tert-butyl methyl ether (2x). The combined organic phases are washed with brine, dried over sodium sulphate and filtered, and the filtrate is concentrated by evaporation. The title compound is obtained from the residue by means of flash chromatography ($SiO_2$ 60F).

General method F: (lactone-opening/silylation)

A solution of 1 mmol of "lactone" in 5 ml of dioxan is treated with 5 ml of water and 1.1 mmol of lithium hydroxide monohydrate. After stirring for 4-6 hours, the reaction mixture is treated with ice-1M citric acid solution and extracted with tert-butyl methyl ether (3x). The combined organic phases are washed with cold water and cold brine, dried over sodium sulphate and filtered, and the filtrate is concentrated by evaporation at room temperature. The residue is immediately dissolved in 8 ml of N,N-dimethylformamide and treated with 5 mmol of tert-butyl-chloro-dimethyl-silane and 8.8 mmol of imidazole. After stirring for 10-20 hours, the reaction mixture is concentrated by evaporation. The residue is taken up in diethyl ether and water, adjusted to pH 4 with 1M citric acid solution and the organic phase is separated. The aqueous phase is extracted with diethyl ether (3x) and the combined organic phases are washed with brine, dried over sodium sulphate and filtered, and the filtrate is concentrated by evaporation. The residue is taken up in 3 ml of tetrahydrofuran and treated sequentially with 3 ml of water and 9 ml of acetic acid. After stirring for 3-4 hours, the reaction mixture is poured onto ice-water and extracted with diethyl ether (2x). The combined organic phases are washed with water and brine, dried over sodium sulphate and filtered, and the filtrate is concentrated by evaporation. The title compound is obtained from the residue by means of flash chromatography ($SiO_2$ 60F).

General method G: (amine-Boc-protection)

A solution of 1 mmol of "amine" in 22 ml of dichloromethane at 0˚C is treated sequentially with 2 mmol of N, N-diisopropylethyl amine and 2 mmol of di-tert-butyl dicarbonate. The reaction mixture is warmed to room temperature and stirred over night. The reaction mixture is poured onto water and the organic phase is separated. The aqueous phase is extracted with dichloromethane (2x) and the combined organic phases are washed with brine, dried over sodium sulphate and filtered, and the filtrate is concentrated by evaporation. The title compound is obtained from the residue by means of flash chromatography ($SiO_2$ 60F).

General method H: (aryl- or heteroaryl-lithium addition to aldehyde)

A solution of 1 mmol of "arylhalide" or "heteroarylhalide" in 2.70 ml of tetrahydrofuran is cooled to -100˚C and treated with 1 mmol of butyllithium solution (1.6M in hexane) at -100˚C. The reaction mixture stirred for 1 minute at -100˚C and quickly added to a solution of 1 mmol of 2-[2-azido-2-(4-isopropyl-5-oxo-tetrahydro-furan-2-yl)-ethyl]-3-methyl-butyraldehyde [173154-02-4] in 1 ml of tetrahydrofuran precooled to -100˚C. The reaction mixture stirred for 15 minutes at -100˚C and quenched with 1M ammonium chloride solution at -100˚C. The reaction mixture is extracted with tert-butyl methyl ether (3x). The combined organic phases are washed with brine, dried over sodium sulphate and filtered, and the filtrate is concentrated by evaporation. The title compound is obtained from the residue by means of flash chromatography ($SiO_2$ 60F).

The following example compounds are prepared as described in detail in WO2005/090305 pages 42 to 47 which description is herewith incorporated.

Example compound 1 K

N-(2-Carbamoyl-2-methylpropyl)-5-amino-4-hydroxy-2-isopropyl-7-[3-(3-methoxypropyl)-1H-indol-5-ylmethyl]-8-methylnonanamide

Example compound 3DD

5-Amino-4-hydroxy-2-isopropyl-7-[3-(3-methoxy-propyl)-1-methyl-1H-indol-5-ylmethyl]-8-methyl-nonanoic acid (5-fluoro-pyridin-2-yl)-amide

Example compound 30VV

5-Amino-4-hydroxy-2-isopropyl-7-[2-(4-methoxy-butyl)-1-oxy-pyridin-4-ylmethyl]-8-methyl-nonanoic acid (tetrahydro-pyran-4-yl)-amide

According to general method B, 284.3 mg [2-hydroxy-1-{2-[2-(4-methoxy-butyl)-1-oxy-pyridin-4-ylmethyl]-3-methyl-butyl}-5-methyl-4-(tetrahydro-pyran-4-ylcarbamoyl)-hexyl]-carbamic acid tert-butyl ester are used to afford the title compound as a yellow oil. Rf = 0.13 (dichloromethane-methanol-25% ammonia conc. 80:10:1); Rt = 3.17 (gradient I).

The starting materials are prepared as follows:

a) [2-Hydroxy-1-{2-[2-(4-methoxy-butyl)-1-oxy-pyridin-4-ylmethyl]-3-methyl-butyl}-5-methyl-4-(tetrahydro-pyran-4-ylcarbamoyl)-hexyl]-carbamic acid tert-butyl ester

A solution of 316.4 mg of [2-hydroxy-1-{2-[2-(4-methoxy-butyl)-pyridin-4-ylmethyl]-3-methylbutyl}-5-methyl-4-(tetrahydro-pyran-4-ylcarbamoyl)-hexyl]-carbamic acid tert-butyl ester in 12 ml of dichloromethane is treated with 270.7 mg of m-chloroperbenzoic acid and stirred for 2 hours at room temperature, poured onto 1 M NaOH and extracted with tert-butyl methyl ether (3x). The combined organic phases are washed with brine, dried over sodium sulphate and filtered, and the filtrate is concentrated by evaporation. From the residue, the title compound is obtained as a colourless glass by means of flash chromatography ($SiO_2$ 60F). Rf = 0.32 (dichloromethane-methanol-25% ammonia conc. 200:15:1); Rt = 4.14 (gradient I).

b) [2-Hydroxy-1-{2-[2-(4-methoxy-butyl)-pyridin-4-ylmethyl]-3-methyl-butyl}-5-methyl-4-(tetrahydro-pyran-4-ylcarbamoyl)-hexyl]-carbamic acid tert-butyl ester

According to general method C, 700 mg of {1-(4-isopropyl-5-oxo-tetrahydro-furan-2-yl)-3-[2-(4-methoxy-butyl)-pyridin-4-ylmethyl]-4-methyl-pentyl}-carbamic acid tert-butyl ester and 560 mg of tetrahydro-pyran-4-ylamine (example VV) are used to afford the title compound as a white foam. Rf = 0.20 (dichloromethane-methanol-25% ammonia conc. 25% 200:10:1); Rt = 3.72 (gradient I).

c) {1-(4-Isopropyl-5-oxo-tetrahydro-furan-2-yl)-3-[2-(4-methoxy-butyl)-pyridin-4-ylmethyl]-4-methyl-pentyl}-carbamic acid tert-butyl ester

According to general method G,2.25 g of 5-{1-amino-3-[2-(4-methoxy-butyl)-pyridin-4-ylmethyl]-4-methyl-pentyl}-3-isopropyl-dihydro-furan-2-one is used to afford the title compound as a yellow oil. Rf = 0.33 (EtOAc-heptane 3:1); Rt = 4.39 (gradient I).

d) 5-{1-Amino-3-[2-(4-methoxy-butyl)-pyridin-4-ylmethyl]-4-methyl-pentyl}-3-isopropyldihydro-furan-2-one

According to Example 1K (steps d-e), 3.28 g of 5-(1-azido-3-{hydroxy-[2-(4-methoxy-butyl)-pyridin-4-yl]-methyl}-4-methyl-pentyl)-3-isopropyl-dihydro-furan-2-one is used to afford the title compound as a colourless oil. Rf = 0.18 (dichloromethane-methanol-25% ammonia conc. 200:10:1); Rt = 2.91 (gradient I).

f) 5-(1-Azido-3-{hydroxy-[2-(4-methoxy-butyl)-pyridin-4-yl]-methyl}-4-methyl-pentyl)-3-isopropyl-dihydro-furan-2-on

According to general method H, 7.0 g of 4-brom-2-(3-methoxy-propyl)-pyridine (residue 30) is used to afford the title compound as an orange oil. Rf = 0.13 (dichloromethane-methanol-25% ammonia conc. 200:10:1); Rt = 4.05 (gradient I).

Residues R[1]:

| | | | |
|---|---|---|---|
| 1 | 2 | 3 | 4 |
| 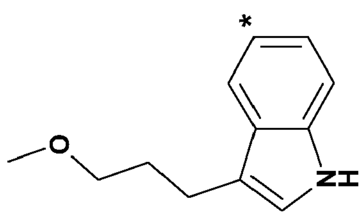 | 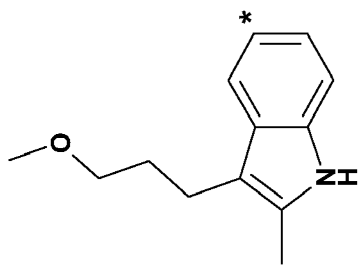 | 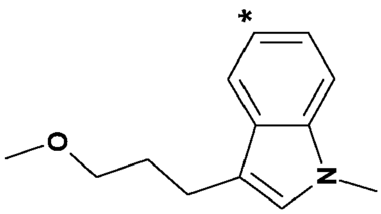 | 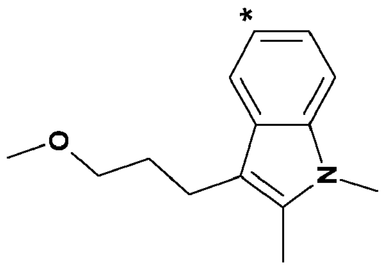 |
| 5 | 6 | 7 | 8 |
| 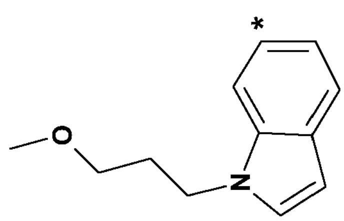 | 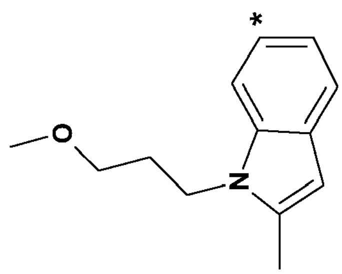 | 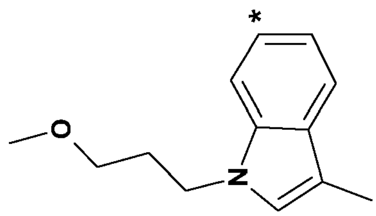 | 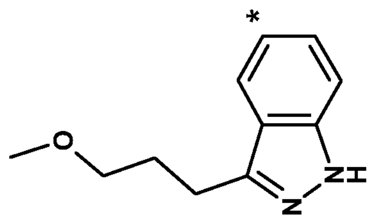 |

(continued)

| | |
|---|---|
| 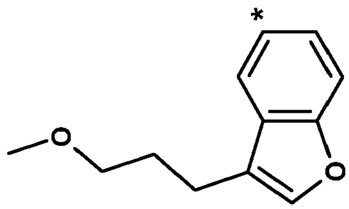 12 | 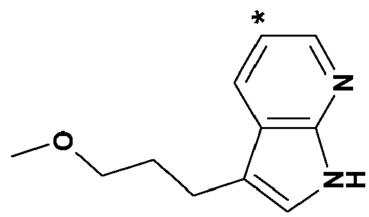 16 |
| 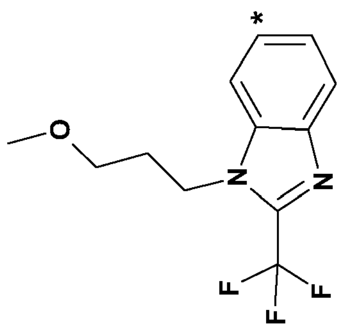 11 | 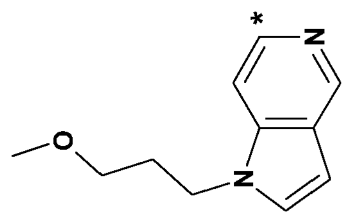 15 |
| 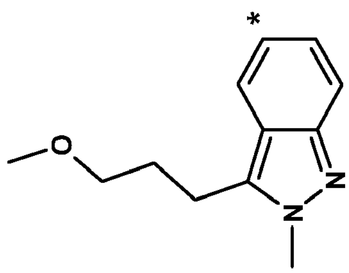 10 | 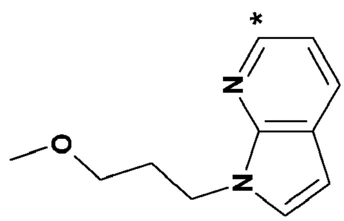 14 |
| 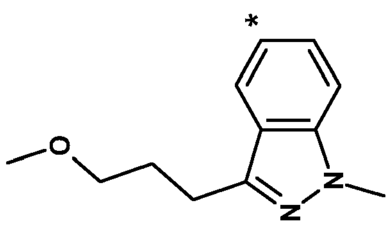 9 | 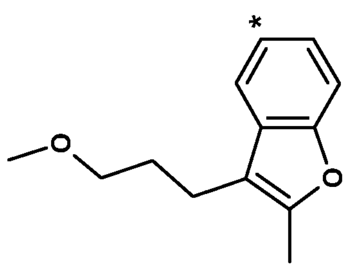 13 |

(continued)

| | | | |
|---|---|---|---|
| 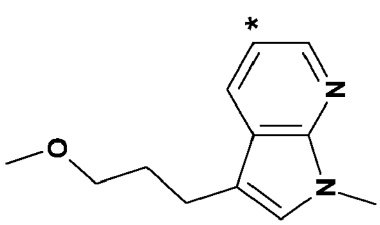 17 | 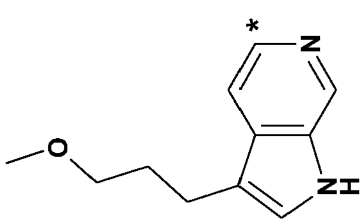 18 | 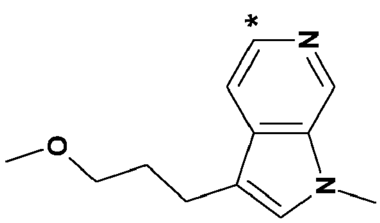 19 | 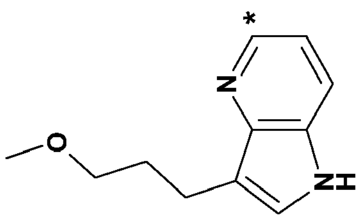 20 |
| 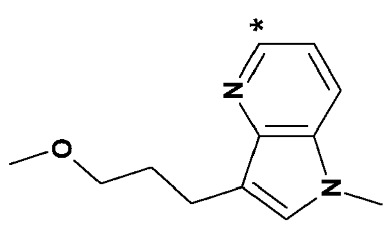 21 | 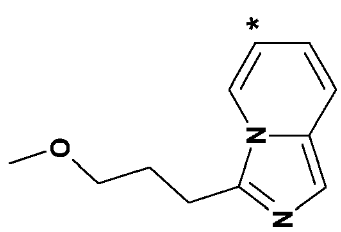 22 | 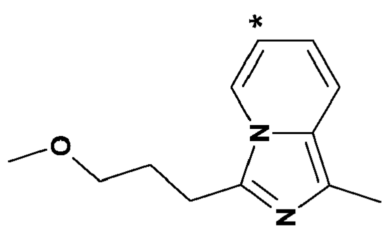 23 | 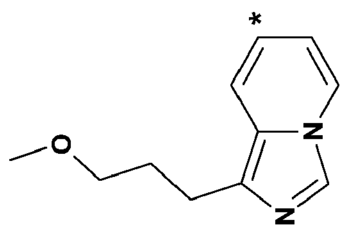 24 |

(continued)

| | |
|---|---|
| 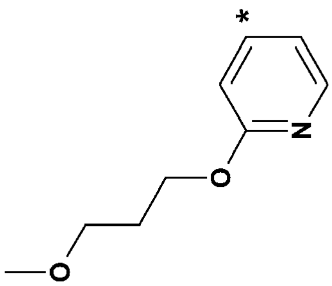 28 | 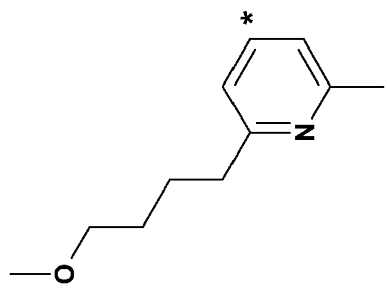 32 |
| 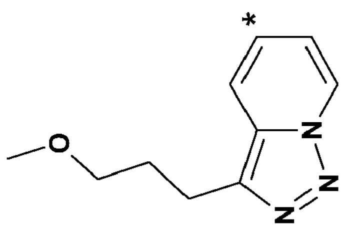 27 | 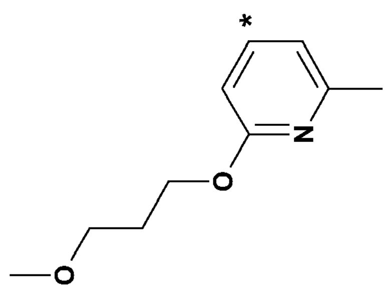 31 |
| 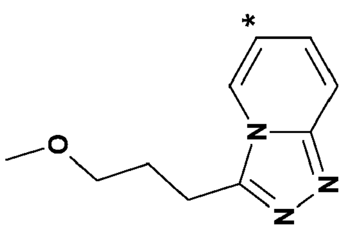 26 | 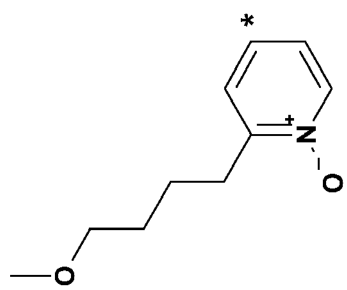 30 |
| 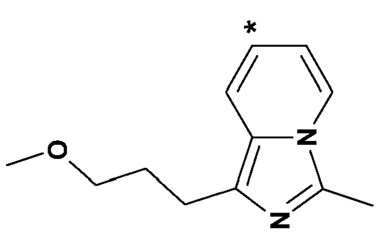 25 | 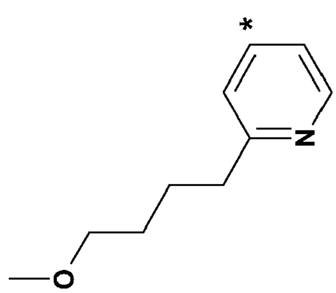 29 |

(continued)

| | | | |
|---|---|---|---|
| 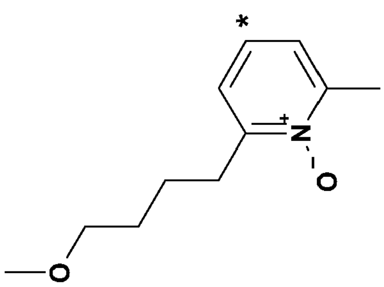 33 | 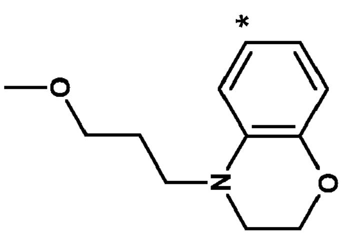 34 | 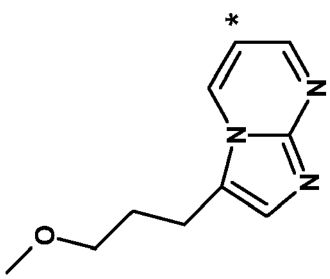 35 | 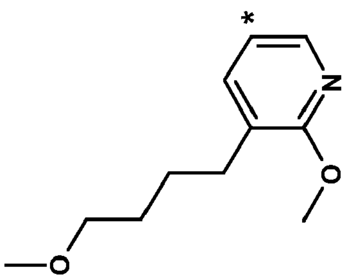 36 |
| 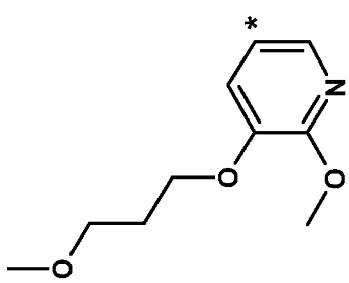 37 | 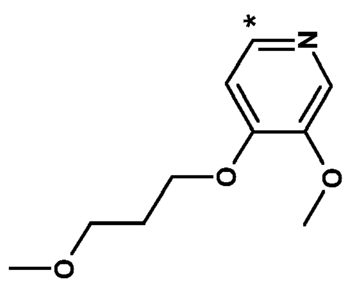 38 | 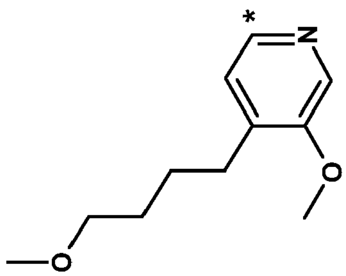 39 | 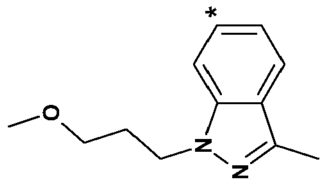 40 |

22 6-Brom-3-(3-methoxypropyl)imidazo[1,5-a]pyridin

A solution of 8.02 g of 3-(6-bromo-imidazo[1,5-a]pyridin-3-yl)-propan-1-ol in 160 ml of N,N-dimethylformamide is treated with 1.38 g of sodium hydride (60% dispersion in oil) and stirred for 1 hour at room temperature. 2.5 ml of methyliodide are added and the reaction mixture is stirred for 2 hours at room temperature. The mixture is poured onto 1M sodium bicarbonate solution and extracted with dichloromethane (3x). The combined organic phases are dried over sodium sulphate and filtered, and the filtrate is concentrated by evaporation. The title compound is obtained from the residue as a yellow oil by means of flash chromatography ($SiO_2$ 60F). Rf = 0.46 (EtOAc); Rt = 2.69 (gradient I).

The starting materials are prepared as follows:

a) 3-(6-Bromo-imidazo[1,5-a]pyridin-3-yl)-propan-1-ol

A solution of 9.62 g of 3-(6-bromo-imidazo[1,5-a]pyridin-3-yl)-propionic acid ethyl ester in 250 ml of tetrahydrofuran at -78°C is treated with 109 ml of DIBAL-H and warmed to room temperature over 1 hour. The mixture is poured onto a mixture of ice and 2M HCl and extracted with ethyl acetate (3x). The combined organic phases are dried over sodium sulphate and filtered, and the filtrate is concentrated by evaporation. The title compound is obtained as a brown oil from the residue by means of flash chromatography ($SiO_2$ 60F). Rf = 0.17 (dichloromethane-methanol-25% ammonia conc. 200:10:1); Rt = 2.19 (gradient I).

b) 3-(6-Bromo-imidazo[1,5-a]pyridin-3-yl)-propionic acid ethyl ester

A solution of 15.59 g of N-(5-bromo-pyridin-2-ylmethyl)-succinamic acid ethyl ester in 170 ml of toluene is treated with 25 ml of phosphor oxychloride and heated for 10 hours to 60°C. Die The solution is cooled to 0°C and slowly treated with 2M NaOH till the ph Value is adjusted to 11. The solution is stirred for 45 minutes at room temperature and extracted with tert-butyl methyl ether (3x). The combined organic phases are dried over sodium sulphate and filtered, and the filtrate is concentrated by evaporation. The title compound is obtained as a yellow solid from the residue by means of flash chromatography ($SiO_2$ 60F). Rf = 0.34 (EtOAc-heptane 1:1); Rt = 2.93 (gradient I).

c) N-(5-Bromo-pyridin-2-ylmethyl)-succinamic acid ethyl ester

A solution of 17.7 g of N-(5-bromo-pyridin-2-ylmethyl)-succinamic acid in 350 ml of ethanol is treated with 15.75 g of camphorsulphonic acid and heated to reflux for 3 hours. The solution is cooled to room temperature and 3 A molecular sieves are added. After 15 Minutes, the mixture is filtered, and the filtrate is concentrated by evaporation. The title compound is obtained as a white solid from the residue by means of flash chromatography ($SiO_2$ 60F). Rf = 0.22 (dichloromethane-methanol-25% ammonia conc. 200:10:1); Rt = 2.90 (gradient I).

d) N-(5-bromo-pyridin-2-ylmethyl)-succinamic acid

A solution of 19.923 g of C-(5-bromo-pyridin-2-yl)-methylamine [173999-23-0] in 34 ml of pyridine is treated with 11.72 g of succinamic anhydride and stirred over night at room temperature. The white solid is collected by filtration, washed with little water and dried. Rt = 2.11 (gradient I).

23 6-Bromo-3-(3-methoxypropyl)-1-methylimidazo[1,5-a]pyridine

According to the method described for Residue 22, the title compound is obtained from 1-(5-bromo-pyridin-2-yl)-ethylamine.

The starting material is prepared as follows:

a) 1-(5-Bromo-pyridin-2-yl)-ethylamine

A solution of 75 g of 5-bromo-pyridin-2-carbonitrile [97483-77-7] in 375 ml of tetrahydrofuran is treated dropwise with a solution of 172 ml methyl magnesium bromide (3M solution in diethyl ether) in 150 ml of tetrahydrofuran and stirred for Minutes at room temperature. The suspension is then treated with 750 ml of methanol and portionwise with 30 g of sodium borohydride. The reaction mixture is stirred 10 hours at room temperature, concentrated by evaporation and treated with ethyl acetate and 2m NaOH. The phases are separated and the aqueous phase is extracted with ethyl acetate (3x). The combined organic phases are washed with brine, dried over sodium sulphate and filtered, and the filtrate is concentrated by evaporation. The title compound is obtained as a brown oil from the residue by means of flash chromatography ($SiO_2$ 60F). Rf = 0.54 (dichloromethane-methanol-25% ammonia conc. 80:10:1); Rt = 2.02 (gradient I).

30 4-Bromo-2-(4-methoxybutyl)pyridin-N-oxid

Example Compounds containing Residue 30 are obtained from precursors to Example Compounds containing Residue 29. In analogy to Example Compound 30VV, the oxidation to the N-oxide is carried out before the last step leading to the final example compound.

32 4-Bromo-2-(4-methoxybutyl)-6-methylpyridine

A solution of 6.54 g 4-(4-bromo-6-methyl-pyridin-2-yl)-butan-1-ol in 75 ml of N,N-dimethylformamide under Ar-atmosphere is cooled to 0˚C. 1.25 g of sodium hydride (60% Dispersion in oil) is added portionwise over 15 minutes. The reaction mixture is stirred for 1 hour at room temperature and treated with 1.90 ml of methyl iodide. The reaction mixture is stirred for 16 hours, poured onto 1M sodium bicarbonate solution and extracted with tert-butyl methyl ether (2x). The combined organic phases are dried over sodium sulphate and filtered, and the filtrate is concentrated by evaporation. The title compound is obtained as a yellow oil from the residue by means of flash chromatography ($SiO_2$ 60F). Rf = 0.28 (EtOAc-heptane 1:1); Rt = 2.45 (gradient I).

The starting materials are prepared as follows:

a) 4-(4-Brom-6-methyl-pyridin-2-yl)-butan-1-ol

A solution of 11.73 g 4-(4-bromo-6-methyl-pyridin-2-yl)-but-3-in-1-ol in 175 ml of ethanol is treated with 7.30 ml of triethylamine and 0.465 g platinum(II)oxide hydrate. The reaction mixture is hydrogenated for 2 hours at room temperature under atmospheric pressure. The reaction mixture is clarified by filtration and the filtrate is concentrated by evaporation. The title compound is obtained as a yellow oil from the residue by means of flash chromatography ($SiO_2$ 60F). Rf = 0.20 (EtOAc-heptane 3:1); Rt = 1.92 (gradient I).

b) 4-(4-Bromo-6-methyl-pyridin-2-yl)-but-3-in-1-ol

A Mixture of 13.85 g of 2,4-dibromo-6-methyl-pyridine [79055-52-0], 1.80 g of bis(triphenylphosphine) palladium (II)chloride and 0.50 g of copper(I)iodide in 330 ml of triethylamine is treated with 4.30 ml of 3-butin-1-ol [927-74-2] under Ar atmosphere at 0˚C. The reaction mixture is stirred for 4 hours at room temperature, diluted with water and extracted with tert-butyl methyl ether (2x). The combined organic phases are dried over sodium sulphate and filtered, and the filtrate is concentrated by evaporation. From the residue, the title compound is obtained as beige solid by re-crystallization from hot ethyl acetate-heptane 3:2. Rf = 0.15 (EtOAc-heptane 2:1); Rt = 2.51 (gradient I).

35 6-Bromo-3-(3-methoxy-propyl)-8-methyl-imidazo[1,2-a]pyridine

A mixture of 0.85 g of 2-amino-5-bromo-3-methyl-pyridine, 0.80 g of 2(S)-chloro-5-methoxy-pentanal and 2.50 ml of ethanol is stirred for 1.5 hours at reflux. The reaction mixture is cooled to room temperature, and poured onto 40 ml of 1M sodium bicarbonate solution and extracted with tert-butyl methyl ether (3x). The combined organic phases are dried over sodium sulphate and filtered, and the filtrate is concentrated by evaporation. The title compound is obtained as a yellow oil from the residue by means of flash chromatography ($SiO_2$ 60F). Rf (EtOAc-heptane 3:1) = 0.29; Rt = 2.69 (gradient I).

The starting material is prepared as follows:

a) 2(S)-Chlor-5-methoxy-pentanal

A solution of 1.46 g of 5-methoxy-pentanal [84629-00-5] in 20 ml of dichloromethane at 0˚C is treated sequentially with 0.118 g of L-proline amid and 1.81 g of N-chlorosuccinimide and stirred for 20 hours at room temperature. 40 ml of hexane are added to the reaction mixture, the mixture is then cooled to 0˚C and filtered. The filtrate is concentrated by evaporation and the residue is purified by short path distillation (bulb to bulb transfer) to afford the title compound as a colourless oil. b.p. ca. 130˚C at 8 mbar.

Residues $NHR^5$:

| | | | |
|---|---|---|---|
| * NHMe A | * NHEt B | * NHPr C | * NHi-Pr D |
| * NHt-Bu E | * NHi-Bu F | 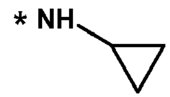 G | 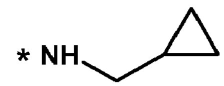 H |
| 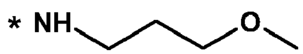 I | 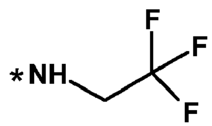 J | 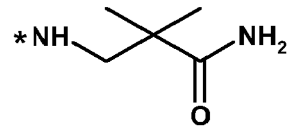 K | 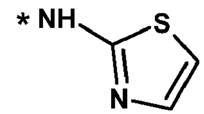 L |
| 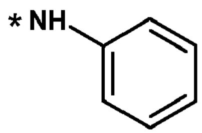 M | 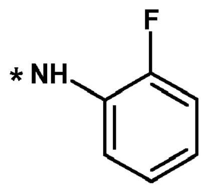 N | 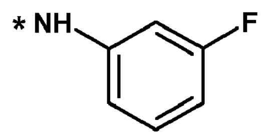 O | 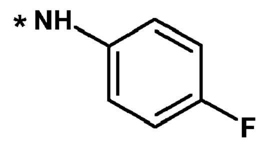 P |
| 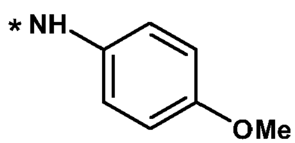 Q | 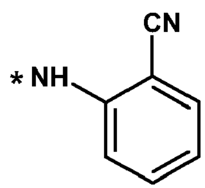 R | 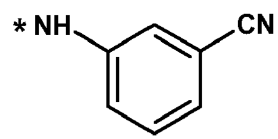 S | 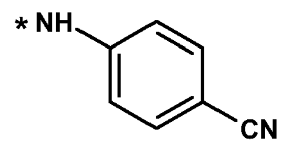 T |

(continued)

| * NHMe A | * NHEt B | * NHPr C | * NHi-Pr D |
|---|---|---|---|
| 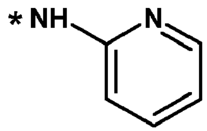 U | 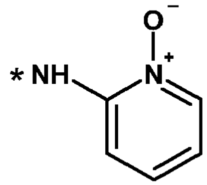 V | 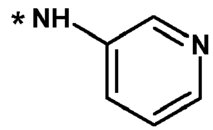 W | 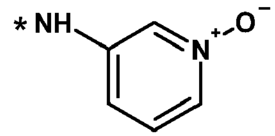 X |
| 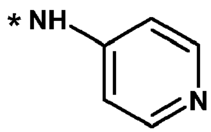 Y | 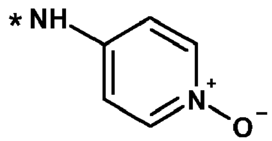 Z | 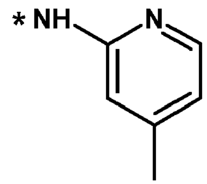 AA | 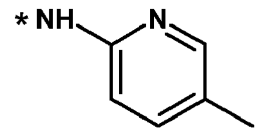 BB |
| 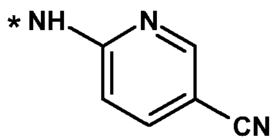 CC | 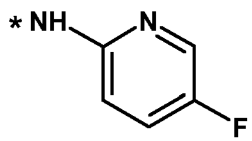 DD | 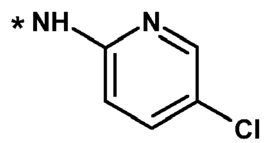 EE | 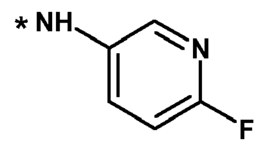 FF |
| 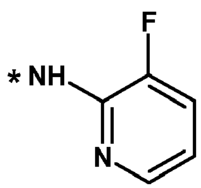 GG | 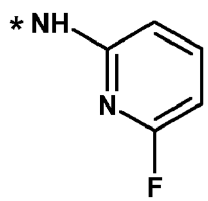 HH | 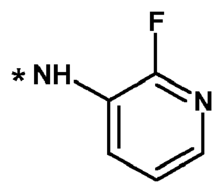 II | 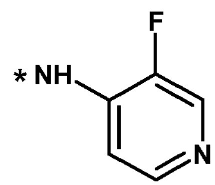 JJ |

(continued)

| * NHMe A | * NHEt B | * NHPr C | * NHi-Pr D |
|---|---|---|---|
| 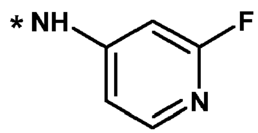 KK | 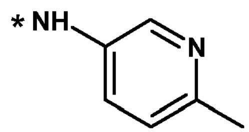 LL | 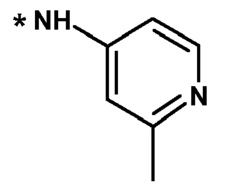 MM | 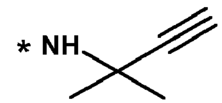 NN |
| 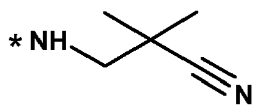 OO | 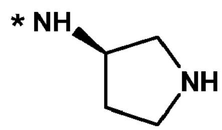 PP | 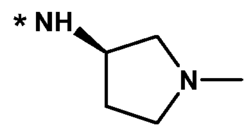 QQ | 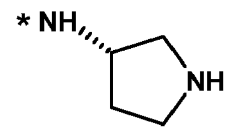 RR |
| 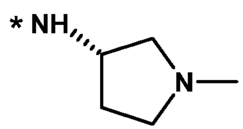 SS | 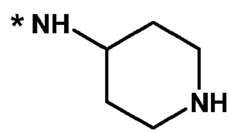 TT | 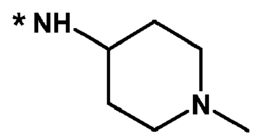 UU | 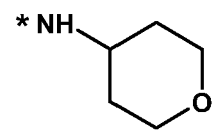 VV |
| 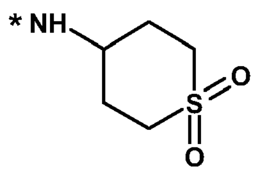 WW | 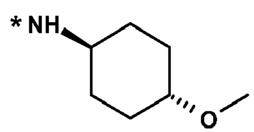 XX | 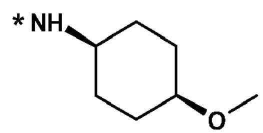 YY | |

The protected or unprotected amines corresponding to above residues $NR^1R^2$ are commercially available and/or prepared according to methods known from the literature.

## Claims

1. Use of a compound of formula

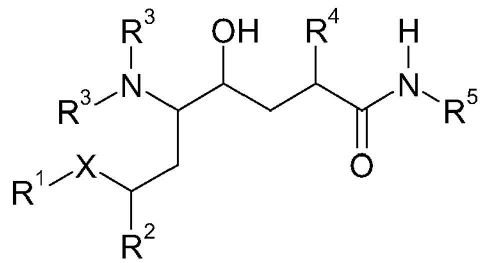

(I)

,

or a pharmaceutically usable salt thereof; wherein

X is $-CH_2-$ ;
(A) $R^1$ is a heterocyclyl radical or a polycyclic, unsaturated hydrocarbon radical which is substituted by one to four radicals selected from $C_1$-$C_6$-alkyl, $C_{3-8}$-cydoalkyl, $C_{3-8}$-cycloalkoxy, $C_{3-8}$-cycloalkoxy-$C_{1-6}$-alkyl, $C_{3-8}$-cycloalkoxy-$C_{1-6}$-alkoxy, $C_1$-$C_6$-alkylamino, di-$C_1$-$C_6$-alkylamino, amino-$C_{1-6}$-alkyl, amino-$C_{2-7}$-alkoxy, polyhalo-$C_{1-6}$-alkyl, polyhalo-$C_{2-7}$-alkoxy, nitro, amino, oxo, oxide, $C_2$-$C_6$-alkenyl, $C_1$-$C_6$-alkoxy, $C_1$-$C_6$-alkanoyloxy, hydroxy, halogen, cyano, carbamoyl, carboxyl,$C_1$-$C_6$-alkylenedioxy, phenyl, phenoxy, phenylthio, phenyl-$C_1$-$C_6$-alkyl or phenyl-$C_1$-$C_6$-alkoxy, pyridylcarbonylamino-$C_{1-6}$-alkyl, $C_{2-7}$-alkenyloxy, $C_{1-6}$-alkoxy-$C_{1-6}$-alkoxy, $C_{1-6}$-alkoxy-$C_{1-6}$-alkoxy-$C_{1-6}$-alkyl, methoxybenzyloxy, hydroxybenzyloxy, methylenedioxybenzyloxy, dioxolanyl-$C_{1-6}$-alkoxy, $C_{3-8}$-cycloalkyl-$C_{1-6}$-alkyl, $C_{3-8}$-cycloalkyl-$C_{1-6}$-alkoxy, hydroxy-$C_{2-7}$-alkoxy, carbamoyloxy-$C_{2-7}$-alkoxy, pyridyl-carbamoyloxy-$C_{2-7}$-alkoxy, benzoyloxy-$C_{2-7}$-alkoxy, $C_{1-6}$-alkoxycarbonyl, $C_{1-6}$-alkylcarbonylamino,$C_{1-6}$-alkylcarbonylamino-$C_{1-6}$-alkyl,$C_{1-6}$-alkylcarbonylamino-$C_{2-7}$-alkoxy,(N-$C_{1-6}$-alkyl)-$C_{1-6}$-alkylcarbonylamino-$C_{1-6}$-alkyl, (N-$C_{1-6}$-alkyl)-$C_{1-6}$-alkylcarbonylamino-$C_{2-7}$-alkoxy, $C_{3-8}$-cycloalkylcarbonylamino-$C_{1-6}$-alkyl, $C_{3-8}$-cycloalkylcarbonylamino-$C_{2-7}$-alkoxy, $C_{1-6}$-alkoxy-$C_{1-6}$-alkyl, hydroxy-$C_{1-6}$-alkyl, hydroxy-$C_{2-7}$-alkoxy-$C_{1-6}$-alkyl, hydroxy-$C_{2-7}$-alkoxy-$C_{1-6}$-alkoxy, $C_{1-6}$-alkoxycarbonylamino-$C_{1-6}$-alkyl, $C_{1-6}$-alkoxycarbonylamino-$C_{2-7}$-alkoxy, $C_{1-6}$-alkylaminocarbonylamino-$C_{1-6}$-alkyl, $C_{1-6}$-alkylaminocarbonyl-amino-$C_{2-7}$-alkoxy, $C_{1-6}$-alkylaminocarbonyl-$C_{1-6}$-alkyl, $C_{1-6}$-alkylaminocarbonyl-$C_{1-6}$-alkoxy, $C_{1-6}$-alkylaminocarbonyl-$C_{1-6}$-alkoxy-$C_{1-6}$-alkyl, di-$C_{1-6}$-alkylaminocarbonyl-$C_{1-6}$-alkyl, di-$C_{1-6}$-alkylaminocarbonyl-$C_{1-6}$-alkoxy, $C_{1-6}$-alkylcarbonyloxy-$C_{1-6}$-alkyl, $C_{1-6}$-alkylcarbonyloxy-$C_{2-6}$-alkoxy, cyano-$C_{1-6}$-alkyl, cyano-$C_{1-6}$-alkoxy, 2-oxooxazolidinyl-$C_{1-6}$-alkyl, 2-oxooxazolidinyl-$C_{1-6}$-alkoxy, $C_{1-6}$-alkoxycarbonyl-$C_{1-6}$-alkyl, $C_{1-6}$-alkoxycarbonyl-$C_{1-6}$-alkoxy, $C_{1-6}$-alkylsulphonylamino-$C_{1-6}$-alkyl, $C_{1-6}$-alkylsulphonylamino-$C_{1-6}$-alkoxy, (N-$C_{1-6}$-Alkyl)-$C_{1-6}$-alkylsulphonylamino-$C_{1-6}$-alkyl, (N-$C_{1-6}$-alkyl)-$C_{1-6}$-alkylsulphonylamino-$C_{2-7}$-alkoxy, $C_{1-6}$-alkylamino-$C_{1-6}$-alkyl, $C_{1-6}$-alkylamino-$C_{2-7}$-alkoxy, di-$C_{1-6}$-alkylamino-$C_{1-6}$-alkyl, di-$C_{1-6}$-alkylamino-$C_{2-7}$-alkoxy, $C_{1-6}$-alkylsulphonyl-$C_{1-6}$-alkyl, $C_{1-6}$-alkylsulphonyl-$C_{1-6}$-alkoxy, carboxy-$C_{1-6}$-alkyl, carboxy-$C_{1-6}$-alkoxy, carboxy-$C_{1-6}$-alkoxy-$C_{1-6}$-alkyl,$C_{1-6}$-alkoxy-$C_{1-6}$-alkylcarbonyl,acyl-$C_{1-6}$-alkoxy-$C_{1-6}$-alkyl,(N-$C_{1-6}$-alkyl)-$C_{1-6}$-alkoxycarbonylamino, (N-hydroxy)-$C_{1-6}$-alkylaminocarbonyl-$C_{1-6}$-alkyl, (N-hydroxy)-$C_{1-6}$-alkylaminocarbonyl-$C_{1-6}$-alkoxy, (N-hydroxy)aminocarbonyl-$C_{1-6}$-alkyl, (N-hydroxy)aminocarbonyl-$C_{1-6}$-alkoxy, $C_{1-6}$-alkoxyaminocarbonyl-$C_{1-6}$-alkyl, 6-alkoxyaminocarbonyl-$C_{1-6}$-alkoxy, (N-$C_{1-6}$-alkoxy)-$C_{1-6}$-alkylaminocarbonyl-$C_{1-6}$-alkyl, (N-$C_{1-6}$-alkoxy)-$C_{1-6}$-alkylaminocarbonyl-$C_{1-6}$-alkoxy, (N-acyl)-$C_{1-6}$-alkoxy-$C_{1-6}$-alkylamino, $C_{1-6}$-alkoxy-$C_{1-6}$-alkylcarbamoyl, (N-$C_{1-6}$-alkyl)-$C_{1-6}$-alkoxy-$C_{1-6}$-alkylcarbamoyl, $C_{1-6}$-alkoxy-$C_{1-6}$-alkylcarbonyl, $C_{1-6}$-alkoxy-$C_{1-6}$-alkylcarbonylamino, (N-$C_{1-6}$-alkyl)-$C_{1-6}$-alkoxy-$C_{1-6}$-alkylcarbonylamino, 1-$C_{1-6}$-alkoxy-$C_{1-6}$-alkylimidazol-2-yl, 1-$C_{1-6}$-alkoxy-$C_{1-6}$-alkyltetrazol-5-yl, 5-$C_{1-6}$-alkoxy-$C_{1-6}$-alkyltetrazol-1-yl, 2-$C_{1-6}$-alkoxy-$C_{1-6}$-alkyl-4-oxoimidazol-1-yl, carbamoyl-$C_{1-6}$-alkyl, carbamoyl-$C_{1-6}$-alkoxy, $C_{1-6}$-alkylcarbamoyl, di-$C_{1-6}$-alkylcarbamoyl, $C_{1-6}$-alkylsulphonyl, $C_{1-6}$-alkylamidinyl, acetamidinyl-$C_{1-6}$-alkyl, O-methyl-oximyl-$C_{1-6}$-alkyl, O,N-dimethylhydroxylamino-$C_{1-6}$-alkyl, $C_{3-6}$-cycloalkyl-$C_{1-6}$-alkanoyl, aryl-$C_{1-6}$-alkanoyl or heterocyclyl-$C_{1-6}$-alkanoyl, each of which is optionally substituted by halogen, $C_1$-$C_6$-alkyl, $C_{1-6}$-alkoxy, hydroxy, $C_1$-$C_6$-alkylamino, di-$C_1$-$C_6$-alkylamino, $C_{1-6}$-alkoxycarbonyl, hydroxy-$C_{1-6}$-alkyl or trifluoromethyl, and also pyridyl, pyridyloxy, pyridylthio, pyridylamino, pyridyl-$C_{1-6}$-alkyl, pyridyl-$C_{1-6}$-alkoxy, pyrimidinyl, pyrimidinyloxy, pyrimidinylthio, py-

rimidinylamino, pyrimidinyl-$C_{1-6}$-alkyl, pyrimidinyl-$C_{1-6}$-alkoxy, thienyl, thienyl-$C_{1-6}$-alkyl, thienyl-$C_{1-6}$-alkoxy, furyl, furyl-$C_{1-6}$-alkyl or furyl-$C_{1-6}$-alkoxy, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-yl-alkoxy, 3-methyl[1,2,4]oxadiazol-5-ylalkyl, 3-methyl[1,2,4]oxadiazol-5-ylalkoxy, 5-methyl [1,2,4]oxadiazol-3-ylalkyl, 5-methyl[1,2,4]oxadiazol-3-ylalkoxy, tetrazol-1-yl-alkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-yl-alkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-$C_{1-6}$-alkoxy-$C_{1-6}$-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl, each of which is optionally substituted by halogen, $C_{1-6}$-alkyl, $C_{1-6}$-alkoxy or dihydroxy-$C_{1-6}$-alkylaminocarbonyl, and the -O-$CH_2CH(OH)CH_2NR_x$ radical where $NR_x$ is a mono- or di-$C_{1-6}$-alkylamino, piperidino, morpholino, piperazino or N-methylpiperazino radical,
whereby, preferably, in the case that $R^1$ is naphthyl or cyclohexenophenyl, at least the ring of said $R^1$ radicals not bonded directly to X is substituted as specified; or
(B) $R^1$ is pyrazinyl, triazolyl, imidazolyl, benzothiazolyl, pyranyl, tetrahydropyranyl, azetidinyl, morpholinyl, quinazolinyl, quinoxalinyl, isoquinolyl, benzo[b]thienyl, isobenzofuranyl, benzimidazolyl, 2-oxobenzimidazolyl, oxazolyl, thiazolyl, pyrrolyl, pyrazolyl, triazinyl, dihydrobenzofuranyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 1,1,3-trioxodihydro-2H-1$\lambda^6$-benzo[1,4]thiazinyl, 1-oxo-pyridyl, dihydro-3H-benzo-[1,4]oxazinyl, 2-oxotetrahydrobenzo[e][1,4]diazepinyl, 2-oxodihydrobenzo-[e][1,4]diazepinyl, 1H-pyrrolizinyl, phthalazinyl, 1-oxo-3H-isobenzofuranyl, 4-oxo-3H-thieno[2,3-d]pyrimidinyl, 3-oxo-4H-benzo[1,4]oxazinyl, [1,5]naphthyridyl, dihydro-2H-benzo[1,4]thiazinyl, 1,1-dioxodihydro-2H-benzo[1,4]thiazinyl, 2-oxo-1H-pyrido-[2,3-b][1,4]oxazinyl, dihydro-1 H-pyrido[2,3-b][1,4]oxazinyl, 1 H-pyrrolo[2,3-b]pyridyl, benzo[1,3]dioxolyl, benzooxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl, or 2-oxotetrahydropyrimidinyl;
$R^2$ is $C_1$-$C_6$-alkyl or $C_3$-$C_6$-cycloalkyl;
$R^3$ are each independently H, $C_1$-$C_6$-alkyl, $C_{1-6}$-alkoxycarbonyl or $C_1$-$C_6$-alkanoyl;
$R^4$ is $C_1$-$C_6$-alkyl, $C_3$-$C_6$-cycloalkyl, $C_2$-$C_6$-alkenyl or unsubstituted or substituted aryl-$C_1$-$C_6$-alkyl; and
$R^5$ is $C_1$-$C_6$-alkyl, $C_1$-$C_6$-hydroxyalkyl, $C_1$-$C_6$-alkoxy-$C_1$-$C_6$-alkyl, $C_1$-$C_6$-alkanoyloxy-$C_1$-$C_6$-alkyl, $C_1$-$C_6$-aminoalkyl, $C_1$-$C_6$-alkylamino-$C_1$-$C_6$-alkyl, $C_1$-$C_6$-dialkylamino-$C_1$-$C_6$-alkyl, $C_1$-$C_6$-alkanoylamido-$C_1$-$C_6$-alkyl, HO(O)C-$C_1$-$C_6$-alkyl, $C_1$-$C_6$-alkyl-O-(O)C-$C_1$-$C_6$-alkyl, $H_2N$-C(O)-$C_1$-$C_6$-alkyl, $C_1$-$C_6$-alkyl-HN-C(O)-$C_1$-$C_6$-alkyl, ($C_1$-$C_6$-alkyl)$_2$N-C(O)-$C_1$-$C_6$-alkyl, $C_2$-$C_8$-alkenyl, $C_2$-$C_8$-alkynyl, cyano-$C_1$-$C_6$-alkyl, halo-$C_1$-$C_6$-alkyl, optionally substituted aryl-$C_0$-$C_6$-alkyl, optionally substituted $C_3$-$C_8$-cydoalkyl-$C_0$-$C_6$-alkyl or optionally substituted heterocyclyl-$C_0$-$C_6$-alkyl,

for the preparation of a medication for the inhibition of beta-secretase, cathepsin D, plasmepsin II and/or HIV-protease.

**2.** Use according to claim I of a compound of the formula (IA)

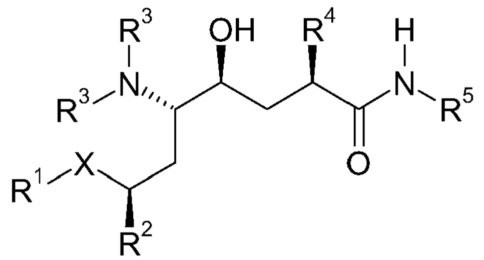

(IA)

,

or a pharmaceutically usable salt thereof; wherein $R^1$, $R^2$, $R^3$, $R^4$, $R^5$ and X are each as defined in Claim 1.

**3.** Use according to claim 1 or 2 of a compound of the formula (I) or a pharmaceutically usable salt thereof or, preferably, of the formula (IA) or a pharmaceutically usable salt thereof, in which at least one, and most preferred all, substituent (s) is (are) defined as follows:

X is $CH_2$;
$R^1$ is as specified for (A) or (B), preferably as specified for (A);
$R^2$ is $C_1$-$C_6$-alkyl;
$R^3$ is H;
$R^4$ is $C_1$-$C_6$-alkyl; and
$R^5$ is $C_1$-$C_6$-alkyl, $C_1$-$C_6$-alkoxy-$C_1$-$C_6$-alkyl, halo-$C_1$-$C_6$-alkyl, $C_2$-$C_8$-alkynyl, cyano-$C_1$-$C_6$-alkyl, optionally substituted $C_3$-$C_6$-cycloalkyl, $C_3$-$C_8$-cycloalkyl-$C_1$-$C_6$-alkyl, optionally substituted aryl, optionally substituted heterocyclyl-$C_0$-$C_6$-alkyl which, for $C_0$-alkyl, is bonded via a carbon atom or $H_2N$-C(O)-$C_1$-$C_6$-alkyl.

**4.** Use according to claim 3, wherein
$R^1$ radicals are selected from benzoimidazolyl, di-$C_{1-6}$-alkoxypyrimidinyl, 2- or 5-benzo[b]thienyl, 6- or 7-isoquinolyl, 6- or 7-tetrahydroquinolyl, 6- or 7-tetrahydroisoquinolyl, 6-quinoxalinyl, 6- or 7-quinazolinyl, dihydro-3H-benzo[1,4] oxazinyl, 3,4-dihydro-2H-benzo[1,4]oxazinyl, 3-oxo-4H-benzo[1,4]oxazinyl, 2-oxobenzooxazolyl, 2-oxo-1,3-dihydroindolyl, 2,3-dihydroindolyl, indazolyl, benzofuranyl, 6- or 7-quinolyl, 6- or 7-isoquinolyl, 6- or 7-tetrahydroquinolyl, oxotetrahydroquinolyl, 6- or 7-tetrahydroisoquinolyl, 6-quinoxalinyl, 6- or 7-quinazolinyl, indolyl, 3-oxo-3,4-dihydro-2H-benzo[1,4]oxazinyl, 2-oxo-2,3-dihydrobenzooxazolyl, 2,3-dihydrobenzothiazinyl, imidazolyl, pyridinyl, pyrrolo [2,3-b]pyridinyl, pyrrolo[3,2-c]pyridinyl, pyrrolo[2,3-c]pyridinyl, pyrrolo[3,2-b]pyridinyl, [1,2,3]triazolo[1,5-a]pyridinyl, [1,2,4]triazolo[4,3-a]pyridinyl, imidazo[1,5-a]pyridinyl, imidazo[1,2-a]pyrimidinyl, naphthyl and cyclohexenophenyl, each of which is substituted by from one to four radicals selected from hydroxy, halogen, oxo, oxide, carbamoyl, carboxyl, cyano, trifluoromethyl, $C_{1-6}$-alkyl, $C_{1-6}$-alkoxy, hydroxy-$C_{1-6}$-alkoxy, $C_{1-6}$-alkoxy-$C_{1-6}$-alkyl, $C_{1-6}$-alkoxy-$C_{1-6}$-alkoxy, di-$C_{1-6}$-alkylamino, 2,3-dihydroxypropoxy, 2,3-dihydroxypropoxy-$C_{1-6}$-alkoxy, 2,3-dimethoxypropoxy, methoxybenzyloxy, hydroxybenzyloxy, phenethyloxy, methylenedioxybenzyloxy, dioxolanyl-$C_{1-6}$-alkoxy, cyclopropyl-$C_{1-6}$-alkoxy, pyridylcarbamoyloxy-$C_{1-6}$-alkoxy, 3-morpholino-2-hydroxypropoxy, benzyloxy-$C_{1-6}$-alkoxy, picolyloxy, $C_{1-6}$-alkoxycarbonyl, $C_{1-6}$-alkoxy-$C_{1-6}$-alkoxy-$C_{1-6}$-alkyl, $C_{1-6}$-alkylcarbonylamino, $C_{1-6}$-alkylcarbonylamino-$C_{1-6}$-alkyl, $C_{1-6}$-alkylcarbonylamino-$C_{1-6}$-alkoxy, (N-$C_{1-6}$-alkyl)-$d_{1-6}$-alkylcarbonylamino-$C_{1-6}$-alkyl, (N-$C_{1-6}$-alkyl)-$C_{1-6}$-alkylcarbonylamino-$C_{1-6}$-alkoxy, $C_{3-6}$-cycloalkylcarbonylamino-$C_{1-6}$-alkyl, $C_{3-6}$-cycloalkyl-carbonylamino-$C_{1-6}$-alkoxy, $C_{1-6}$-alkoxy-$C_{1-6}$-alkyl, hydroxy-$C_{1-6}$-alkyl, hydroxy-$C_{1-6}$-alkoxy-$C_{1-6}$-alkyl, hydroxy-$C_{1-6}$-alkoxy-$C_{1-6}$-alkoxy, $C_{1-6}$-alkoxycarbonylamino-$C_{1-6}$-alkyl, $C_{1-6}$-alkoxycarbonylamino-$C_{1-6}$-alkoxy, $C_{1-6}$-alkylaminocarbonylamino-$C_{1-6}$-alkyl, $C_{1-6}$-alkylaminocarbonylamino-$C_{1-6}$-alkoxy, $C_{1-6}$-alkylaminocarbonyl-$C_{1-6}$-alkyl, $C_{1-6}$-alkylaminocarbonyl-$C_{1-6}$-alkoxy, $C_{1-6}$-alkylaminocarbonyl-$C_{1-6}$-alkoxy-$C_{1-6}$-alkyl, di-$C_{1-6}$-alkylaminocarbonyl-$C_{1-6}$-alkyl, di-$C_{1-6}$-alkylaminocarbonyl-$C_{1-6}$-alkoxy, $C_{1-6}$-alkylcarbonyloxy-$C_{1-6}$-alkyl, $C_{1-6}$-alkylcarbonyloxy-$C_{1-6}$-alkoxy, cyano-$C_{1-6}$-alkyl, cyano-$C_{1-6}$-alkoxy, 2-oxooxazolidinyl-$C_{1-6}$-alkyl, 2-oxooxazolidinyl-$C_{1-6}$-alkoxy, $C_{1-6}$-alkoxycarbonyl-$C_{1-6}$-alkyl, $C_{1-6}$-alkoxycarbonyl-$C_{1-6}$-alkoxy, $C_{1-6}$-alkylsulphonylamino-$C_{1-6}$-alkyl, $_{1-6}$-alkylsulphonylamino-$C_{1-6}$-alkoxy, (N-$C_{1-6}$-alkyl)-$C_{1-6}$-alkylsulphonylamino-$C_{1-6}$-alkyl, (N-$C_{1-6}$-alkyl)-$C_{1-6}$-alkylsulphonylamino-$C_{1-6}$-alkoxy, $C_{1-6}$-alkylamino-$C_{1-6}$-alkyl, $C_{1-6}$-alkylamino-$C_{1-6}$-alkoxy, di-$C_{1-6}$-alkylamino-$C_{1-6}$-alkyl, di-$C_{1-6}$-alkylamino-$C_{1-6}$-alkoxy, $C_{1-6}$-alkylsulphonyl-$C_{1-6}$-alkyl, $C_{1-6}$-alkylsulphonyl-$C_{1-6}$-alkoxy, carboxy-$C_{1-6}$-alkyl, carboxy-$C_{1-6}$-alkoxy, carboxy-$C_{1-6}$-alkoxy-$C_{1-6}$-alkyl, $C_{1-6}$-alkoxy-$C_{1-6}$-alkylcarbonyl, acyl-$C_{1-6}$-alkoxy-$C_{1-6}$-alkyl, (N-$C_{1-6}$-alkyl)-$C_{1-6}$-alkoxycarbonylamino, (N-hydroxy)-$C_{1-6}$-alkylaminocarbonyl-$C_{1-6}$-alkyl, (N-hydroxy)-$C_{1-6}$-alkylaminocarbonyl-$C_{1-6}$-alkoxy, (N-hydroxy)aminocarbonyl-$C_{1-6}$-alkyl, (N-hydroxy)aminocarbonyl-$C_{1-6}$-alkoxy, $C_{1-6}$-alkoxyaminocarbonyl-$C_{1-6}$-alkyl, 6-alkoxyaminocarbonyl-$C_{1-6}$-alkoxy, (N-$C_{1-6}$-alkoxy)-$C_{1-6}$-alkylaminocarbonyl-

$C_{1-6}$-alkyl, (N-$C_{1-6}$-alkoxy)-$C_{1-6}$-alkylaminocarbonyl-$C_{1-6}$-alkoxy, (N-acyl)-$C_{1-6}$-alkoxy-$C_{1-6}$-alkylamino, $C_{1-6}$-alkoxy-$C_{1-6}$-alkylcarbamoyl, (N-$C_{1-6}$-alkyl)-$C_{1-6}$-alkoxy-$C_{1-6}$-alkylcarbamoyl, $C_{1-6}$-alkoxy-$C_{1-6}$-alkylcarbonyl, $C_{1-6}$-alkoxy-$C_{1-6}$-alkylcarbonylamino, (N-$C_{1-6}$-alkyl)-$C_{1-6}$-alkoxy-$C_{1-6}$-alkylcarbonylamino, 1-$C_{1-6}$-alkoxy-$C_{1-6}$-alkylimidazol-2-yl, 1-$C_{1-6}$-alkoxy-$C_{1-6}$-alkyltetrazol-5-yl, 5-$C_{1-6}$-alkoxy-$C_{1-6}$-alkyltetrazol-1-yl, 2-$C_{1-6}$-alkoxy-$C_{1-6}$-alkyl-4-oxoimidazol-1-yl, carbamoyl-$C_{1-6}$-alkyl, carbamoyl-$C_{1-6}$-alkoxy, $C_{1-6}$-alkylcarbamoyl, di-$C_{1-6}$-alkylcarbamoyl, $C_{1-6}$-alkylsulphonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-ylalkoxy, 3-methyl[1,2,4]oxadiazol-5-ylalkyl, 3-methyl[1,2,4]oxadiazol-5-yl-alkoxy, 5-methyl[1,2,4]oxadiazol-3-ylalkyl, 5-methyl[1,2,4]oxadiazol-3-ylalkoxy, tetrazol-1-yl-alkyl, tetrazol-1-ylalkoxy, tetrazol-2-ylalkyl, tetrazol-2-ylalkoxy, tetrazol-5-ylalkyl, tetrazol-5-yl-alkoxy, 5-methyltetrazol-1-ylalkyl, 5-methyltetrazol-1-ylalkoxy, thiazol-4-ylalkyl, thiazol-4-yl-alkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-$C_{1-6}$-alkoxy-$C_{1-6}$-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl and 2-oxotetrahydropyrimidinyl, where, in the case of naphthyl, or cyclohexenophenyl, at least the ring system not bonded to X is substituted as specified.

**5.** Method for the inhibition of beta-secretase, cathepsin D, plasmepsin II and/or HIV-protease consisting of the application of a therapeutically effective dose of a compound of the general formula (I) or (IA) or a pharmaceutically usable salt thereof according to the claims 1 to 4.

**6.** Use of a compound of the general formula (I) and (IA) or a pharmaceutically usable salt thereof, according to any one of the claims 1 and 4 for the preparation of a medication for the prevention, delay of progression or treatment of Alzheimer Disease., malaria or HIV infection.

European Patent Office

# PARTIAL EUROPEAN SEARCH REPORT

which under Rule 45 of the European Patent Convention shall be considered, for the purposes of subsequent proceedings, as the European search report

Application Number EP 06 12 0678

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/103653 A1 (ELAN PHARM INC [US]; JOHN VARGHESE [US]; MAILLARD MICHEL [US]) 18 December 2003 (2003-12-18)<br>See page 7, line 5, 6 and page 14, line 20, 21: treatment of Alzheimer<br>See page 13, lines 1-16 and page 18, lines 6-29 and pages 320-321, examples E and F: inhibition of beta-secretase<br>See compounds having Registry Number: 173333-98-7, 173334-39-9, 173335-64-3, 173335-65-4, 173335-66-5, 173335-67-6, 173398-84-0, 173399-30-9, 173399-68-3, 173521-17-0<br>* claim 1 *<br>See compound of page 129, example 4 and compounds in page 337, lines 16-26<br>-----<br>-/-- | 1-6 | INV.<br>A61K31/404<br>A61P25/28<br>A61P31/18<br>A61P33/06 |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 22 January 2007 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C07)

European Patent Office

# PARTIAL EUROPEAN SEARCH REPORT

Application Number
EP 06 12 0678

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/051895 A1 (NOVARTIS AG [CH]; NOVARTIS PHARMA GMBH [AT]; BAESCHLIN DANIEL KASPAR [) 9 June 2005 (2005-06-09)<br>See page 28, line 16, page 29, line 23 and page 31, line 19: Alzheimer<br>* page 31, line 19 *<br>* claims; examples *<br>See claim 1, page 124, lines 15 and 16 and claim 19, page 128, lines 29-30: option where R3 and R4 form a carbocyclic or an heterocyclic ring.<br>----- | 1-4,6 | |
| A | EP 0 678 503 A1 (CIBA GEIGY AG [CH] NOVARTIS AG [CH]; NOVARTIS ERFIND VERWALT GMBH [AT]) 25 October 1995 (1995-10-25)<br>* page 97, lines 24,25 *<br>See compounds having Registry Number: 173333-98-7, 173334-39-9, 173335-64-3, 173335-65-4, 173335-66-5, 173335-67-6, 173398-84-0, 173399-30-9, 173399-68-3, 173521-17-0<br>* page 10, line 47 - page 11, line 2 *<br>* page 11, lines 47-52 *<br>----- | 1-6 | TECHNICAL FIELDS SEARCHED (IPC) |
| P,D, A | WO 2005/090305 A (SPEEDEL EXPERIMENTA AG [CH]; HEROLD PETER [CH]; STUTZ STEFAN [CH]; MAH) 29 September 2005 (2005-09-29)<br>* page 1, lines 1-2 *<br>* page 15, paragraph 4 *<br>* page 17, lines 19-22 *<br>* claims 1,10; examples *<br>----- | 1-6 | |
| A | ALLIKMETS K: "ALISKIREN SPEEDEL" CURRENT OPINION IN INVESTIGATIONAL DRUGS, PHARMAPRESS, US,<br>vol. 3, no. 10, 2002, pages 1479-1482, XP009017210<br>ISSN: 1472-4472<br>* the whole document *<br>----- | 1-6 | |

4

EPO FORM 1503 03.82 (P04C10)

European Patent Office

**INCOMPLETE SEARCH SHEET C**

Application Number
EP 06 12 0678

Although claim 5 is directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 06 12 0678

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2007

| Patent document cited in search report | Publication date | Patent family member(s) | Publication date |
|---|---|---|---|
| WO 03103653 A1 | 18-12-2003 | AU 2003238007 A1 | 22-12-2003 |
| WO 2005051895 A1 | 09-06-2005 | AU 2004293178 A1 | 09-06-2005 |
| | | CN 1882528 A | 20-12-2006 |
| | | EP 1689702 A1 | 16-08-2006 |
| | | KR 20060111530 A | 27-10-2006 |
| EP 0678503 A1 | 25-10-1995 | AT 183997 T | 15-09-1999 |
| | | AU 699616 B2 | 10-12-1998 |
| | | AU 1642195 A | 26-10-1995 |
| | | CA 2147056 A1 | 19-10-1995 |
| | | CN 1117960 A | 06-03-1996 |
| | | CN 1550491 A | 01-12-2004 |
| | | CY 2208 A | 08-11-2002 |
| | | CZ 9500976 A3 | 15-11-1995 |
| | | DE 59506707 D1 | 07-10-1999 |
| | | DK 678503 T3 | 20-03-2000 |
| | | ES 2137478 T3 | 16-12-1999 |
| | | FI 951771 A | 19-10-1995 |
| | | GR 3031997 T3 | 31-03-2000 |
| | | HU 74074 A2 | 28-10-1996 |
| | | HU 71701 A2 | 29-01-1996 |
| | | IL 113403 A | 24-07-2001 |
| | | JP 3240322 B2 | 17-12-2001 |
| | | JP 8081430 A | 26-03-1996 |
| | | NO 951441 A | 19-10-1995 |
| | | NZ 270936 A | 24-06-1997 |
| | | TW 402582 B | 21-08-2000 |
| | | US 5559111 A | 24-09-1996 |
| | | ZA 9503050 A | 08-11-1995 |
| | | ZA 9503051 A | 18-10-1995 |
| | | ZA 9503052 A | 18-10-1995 |
| WO 2005090305 A | 29-09-2005 | AR 048320 A1 | 19-04-2006 |
| | | CA 2560199 A1 | 29-09-2005 |
| | | EP 1699762 A1 | 13-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 580000 A
- EP 678503 A
- WO 0109079 A
- WO 0109083 A
- WO 0202487 A
- WO 0202500 A
- WO 0202508 A
- WO 0208172 A
- WO 02092828 A
- WO 2005090305 A


### Non-patent literature cited in the description


- **VERDILE et al.** *Pharmacol. Res,* 2004, vol. 50, 397-409
- **THOMPSON et al.** *Curr. Pharm. Des.,* 2005, vol. 11, 3383-3404
- **DAVIDSON et al.** *J. Neurol. Neurosurg. Psychiatry,* 2006, vol. 77, 515-517
- **COOMBS et al.** *Trends Parasitol,* 2001, vol. 17, 532-537
- **ANDREWS et al.** *Antimicrob. Agents Chemother,* 2006, vol. 50, 639-648
- **RANA et al.** *Pharmacotherapy,* 1999, vol. 19, 35-59
- **MORSE et al.** *Lancet Infect. Dis.,* 2006, vol. 6, 215-225
- *Helvetica Chemica Acta,* 2003, vol. 86, 2848-2870